(19) Europäisches Patentamt / European Patent Office / Office européen des brevets

(11) **EP 4 471 135 A1**

(12) **EUROPEAN PATENT APPLICATION**
published in accordance with Art. 153(4) EPC

(43) Date of publication:
04.12.2024 Bulletin 2024/49

(21) Application number: 23745039.0

(22) Date of filing: 23.01.2023

(51) International Patent Classification (IPC):
$C12N\ 9/02^{(2006.01)}$    $C12M\ 1/34^{(2006.01)}$
$C12Q\ 1/26^{(2006.01)}$    $G01N\ 27/327^{(2006.01)}$
$G01N\ 27/416^{(2006.01)}$

(52) Cooperative Patent Classification (CPC):
C12M 1/34; C12N 9/0004; C12Q 1/26;
G01N 27/327; G01N 27/416

(86) International application number:
PCT/JP2023/001930

(87) International publication number:
WO 2023/145689 (03.08.2023 Gazette 2023/31)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR

(30) Priority: 27.01.2022 JP 2022011010

(71) Applicant: Provigate Inc.
Tokyo 113-0033 (JP)

(72) Inventors:
• SHINOHARA Takeshi
  Tokyo 113-0033 (JP)
• KATAYAMA Norikazu
  Tokyo 113-0033 (JP)

• MINOURA Itsushi
  Tokyo 113-0033 (JP)
• TAKEDA Kein
  Tokyo 113-0033 (JP)

(74) Representative: Michalski Hüttermann & Partner
Patentanwälte mbB
Kaistraße 16A
40221 Düsseldorf (DE)

Remarks:
The complete document including Reference
Table(s) and the Sequence Listing(s) can be
downloaded from the EPO website

(54) **FRUCTOSYL AMINO ACID OXIDASE, METHOD FOR PRODUCING FRUCTOSYL AMINO ACID OXIDASE, GLYCATED PROTEIN SENSOR, AND METHOD FOR MEASURING GLYCATED PROTEIN**

(57) [Object] For a glycated protein sensor provided with an immobilized enzyme, a technology for increasing measurement accuracy and the stability of the glycated protein sensor is essential. Unfortunately, there are no known enzymes that can increase the accuracy and stability of measurements that use a glycated protein sensor. [Solution] The present invention provides a fructosyl amino acid oxidase, and a residual activity exhibited by the fructosyl amino acid oxidase after being treated at a temperature of 65°C to 80°C for 20 minutes while being immobilized on a support is 70% or greater. The present invention also provides a glycated protein sensor and a method for measuring a glycated protein.

FIG. 3

EP 4 471 135 A1

**Description**

TECHNICAL FIELD

The present invention relates to a fructosyl amino acid oxidase, to a method for producing a fructosyl amino acid oxidase, to a glycated protein sensor including a fructosyl amino acid oxidase immobilized on a support, and to a method for measuring a glycated protein including detecting a glycated protein by using a reaction of a fructosyl amino acid oxidase immobilized on a support.

BACKGROUND ART

Measurements of glycated proteins are performed to diagnose diabetes or to provide a management index for controlling blood sugar. For example, glycated hemoglobin and glycated albumin are frequently measured in clinical settings. Examples of known methods for measuring a glycated protein include electrophoresis, high-performance liquid chromatography, immunoassay, and enzymatic analysis.

A method for measuring a glycated protein by enzymatic analysis is a method in which, in a first step, a protein is degraded into amino acids or peptides by using a protease; in a second step, among the amino acids or peptides, an amino acid that has been glycated (hereinafter also referred to as a "glycated amino acid" or a "fructosyl amino acid") or a peptide containing a glycated amino acid (hereinafter also referred to as a "glycated peptide" or a "fructosyl peptide") is reacted with a fructosyl amino acid oxidase to generate hydrogen peroxide; and, in a third step, the hydrogen peroxide is converted into a color development reaction to measure an absorbance, or electrons emitted as a result of the decomposition of the hydrogen peroxide at an electrode are detected (see Patent Literature 1).

There are known inventions related to reagents or methods for measuring a glycated protein that are characterized by a variant of a fructosyl amino acid oxidase or by a fructosyl amino acid oxidase so that accurate measurement of a particular glycated amino acid or glycated peptide can be achieved. Some known examples are as follows: a fructosyl amino acid oxidase that consists of an amino acid sequence obtained by modifying the amino acid sequence of a fructosyl amino acid oxidase of the genus Coniochaeta and which acts on fructosyl valyl histidine and substantially does not act on other glycated amino acids (see Patent Literature 2 and 3); a method for measuring hemoglobin A1c by using a reaction of a fructosyl amino acid oxidase that has a property of having a reactivity with fructosyl lysine of 30% or less based on its reactivity with fructosyl valyl histidine (see Patent Literature 4); a reagent composition for measuring a glycated protein, the composition containing a fructosyl amino acid oxidase that has a feature of a reactivity with fructosyl lysine of 12 or less based on its activity value for fructosyl valyl histidine taken as 100 (see Patent Literature 5); a measurement reagent composition containing an enzyme that is derived from a fructosyl amino acid oxidase of the genus Coniochaeta or the like and which has a reactivity with fructosyl valine of 5% or less based on its reactivity with fructosyl lysine taken as 100% (see Patent Literature 6); a method for measuring glycated hemoglobin including a fructosyl amino acid oxidase derived from the genus Coniochaeta or a modified enzyme of the fructosyl amino acid oxidase, the fructosyl amino acid oxidase having a reactivity with fructosyl lysine significantly reduced with respect to its reactivity with fructosyl valyl histidine over a wide pH range (see Patent Literature 7); and a method for measuring fructosyl lysine that uses a fructosyl amino acid oxidase of Aspergillus oryzae, the fructosyl amino acid oxidase being one that acts on fructosyl lysine and substantially does not act on fructosyl valine (see Patent Literature 8).

CITATION LIST

Patent Literature

PTL 1: International Publication No. 2019/221264
PTL 2: Japanese Unexamined Patent Application Publication No. 2014-183786
PTL 3: International Publication No. 2012/018094
PTL 4: Japanese Unexamined Patent Application Publication No. 2011-229526
PTL 5: Japanese Unexamined Patent Application Publication No. 2010-233502
PTL 6: Japanese Patent No. 6504586
PTL 7: Japanese Unexamined Patent Application Publication No. 2013-176351
PTL 8: Japanese Unexamined Patent Application Publication No. 2009-000084

## SUMMARY OF INVENTION

### TECHNICAL PROBLEM

A first object of the present invention is to provide a novel fructosyl amino acid oxidase.

A second object of the present invention is to provide a fructosyl amino acid oxidase having excellent physicochemical properties.

A third object of the present invention is to provide an enzyme that contributes to measurement accuracy and stability for a glycated protein sensor provided with an immobilized enzyme.

A fourth object of the present invention is to provide a glycated protein sensor including a novel fructosyl amino acid oxidase and/or to provide a method for measuring a glycated protein that uses the sensor.

### SOLUTION TO PROBLEM

The present inventors discovered novel fructosyl amino acid oxidases among genes of unknown function. In addition, the present inventors found that some of the fructosyl amino acid oxidases have excellent physicochemical properties, and further, the present inventors succeeded in imparting the physicochemical properties to other fructosyl amino acid oxidases.

That is, the present invention provides a wild-type fructosyl amino acid oxidase. Furthermore, another aspect of the present invention provides a fructosyl amino acid oxidase having an amino acid sequence modified to confer excellent physicochemical properties for the measurement of glycated proteins and also provides a method for producing the fructosyl amino acid oxidase. Yet another aspect of the present invention provides a glycated protein sensor including such a fructosyl amino acid oxidase and also provides a method for measuring a glycated protein.

Specifically, a fructosyl amino acid oxidase of the present invention is a fructosyl amino acid oxidase that satisfies one or more selected from (1) to (5) described below:

(1) the fructosyl amino acid oxidase comprises an amino acid sequence set forth in SEQ ID NO: 1 or an amino acid sequence identical to the amino acid sequence set forth in SEQ ID NO: 1 except that one or more amino acids are deleted, substituted, added, and/or inserted;

(2) the fructosyl amino acid oxidase comprises an amino acid sequence that has a homology of 30% or greater to the amino acid sequence described in (1) and which satisfies the conditions described below in (2-1) and (2-2);

(2-1) in an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines, and
(2-2) in an instance where the amino acid sequence and the amino acid sequence set forth in SEQ ID NO: 1 are aligned with each other, amino acids at positions in the amino acid sequence that correspond to positions 430 and/or 443 of the amino acid sequence set forth in SEQ ID NO: 1 are lysines;

(3) the fructosyl amino acid oxidase comprises an amino acid sequence of any one of SEQ ID NOs: 2 to 23 or an amino acid sequence identical to the amino acid sequence of any one of SEQ ID NOs: 2 to 23 except that one or more amino acids are deleted, substituted, added, and/or inserted;
(4) a residual activity exhibited by the fructosyl amino acid oxidase after being treated at a temperature of 48°C while being immobilized on a support is higher than a residual activity exhibited by the fructosyl amino acid oxidase after being treated under the same condition but without being immobilized on a support; and
(5) a residual activity exhibited by the fructosyl amino acid oxidase after being treated at a temperature of 65°C to 80°C for 20 minutes while being immobilized on a support is 70% or greater.

Furthermore, the present invention may be a fructosyl amino acid oxidase that satisfies one or more selected from (1) to (3) and satisfies (4) and/or (5).

Furthermore, regarding (4) and/or (5), the present invention may be a fructosyl amino acid oxidase that is immobilized on the support as a result of crosslinking induced by an amine-reactive crosslinking agent.

A fructosyl amino acid oxidase of the present invention may be a recombinant protein.

Another aspect of the present invention is a fructosyl amino acid oxidase that satisfies (2) and further satisfies one or more selected from (2-3) and (2-4) described below:

(2-3) in an instance where the amino acid sequence and the amino acid sequence set forth in SEQ ID NO: 1 are aligned with each other, amino acids at positions in the amino acid sequence that correspond to positions 309 and/or 413 of the amino acid sequence set forth in SEQ ID NO: 1 are lysines; and

(2-4) in an instance where the amino acid sequence and the amino acid sequence set forth in SEQ ID NO: 1 are aligned with each other, an amino acid at a position in the amino acid sequence that corresponds to position 424 of the amino acid sequence set forth in SEQ ID NO: 1 is a lysine.

Another aspect of the present invention is a fructosyl amino acid oxidase that satisfies (2) and further satisfies one or more selected from (2-5) and (2-6) described below:

(2-5) in an instance where the amino acid sequence and the amino acid sequence set forth in SEQ ID NO: 1 are aligned with each other, amino acids at positions in the amino acid sequence that correspond to positions 424, 430, and 443 of the amino acid sequence set forth in SEQ ID NO: 1 are lysines; and

(2-6) in an instance where the amino acid sequence and the amino acid sequence set forth in SEQ ID NO: 1 are aligned with each other, amino acids at positions in the amino acid sequence that correspond to positions 309 and 413 of the amino acid sequence set forth in SEQ ID NO: 1 are lysines.

Furthermore, the present invention may be a fructosyl amino acid oxidase that further satisfies (4).

Furthermore, regarding (4), the present invention may be a fructosyl amino acid oxidase that is immobilized on the support as a result of crosslinking induced by an amine-reactive crosslinking agent.

The amino acid sequences of fructosyl amino acid oxidases of the present invention have a high homology to the amino acid sequence set forth in SEQ ID NO: 1, and specifically, the homology may be 74% or greater.

Another aspect of the present invention is a fructosyl amino acid oxidase that satisfies (2-3) and (2-4), and in an instance where the amino acid sequence and the amino acid sequence set forth in SEQ ID NO: 1 are aligned with each other, amino acids at positions in the amino acid sequence that correspond to positions 34, 177, 233, 234, and 297 of the amino acid sequence set forth in SEQ ID NO: 1 are lysines.

The amino acid sequences of fructosyl amino acid oxidases of the present invention have a high homology to the amino acid sequence set forth in SEQ ID NO: 1, and specifically, the homology may be 74% or greater.

Another aspect of the present invention is a fructosyl amino acid oxidase comprising an amino acid sequence that has a homology of 30% or greater to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines, and the fructosyl amino acid oxidase satisfies (6-1) and/or (6-2) described below:

(6-1) in an instance where the amino acid sequence and the amino acid sequence set forth in SEQ ID NO: 1 are aligned with each other, amino acids at positions in the amino acid sequence that correspond to positions 64, 84, 87, 128, 157, 166, 167, 394, and 397 of the amino acid sequence set forth in SEQ ID NO: 1 are lysines; and

(6-2) in an instance where the amino acid sequence and the amino acid sequence set forth in SEQ ID NO: 1 are aligned with each other, amino acids at positions in the amino acid sequence that correspond to positions 112, 137, 152, 247, 254, 255, 267, 277, and 438 of the amino acid sequence set forth in SEQ ID NO: 1 are lysines.

Another aspect of the present invention is a fructosyl amino acid oxidase comprising an amino acid sequence that has a homology of 30% or greater to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines, and the fructosyl amino acid oxidase includes amino acid substitutions as described below in (7-1) and/or (7-2):

(7-1) in an instance where the amino acid sequence and the amino acid sequence set forth in SEQ ID NO: 1 are aligned with each other, and an amino acid other than lysine is present at a position in the amino acid sequence while a lysine is present at a corresponding position in the amino acid sequence set forth in SEQ ID NO: 1, the amino acid other than lysine is substituted with a lysine; and

(7-2) in an instance where the amino acid sequence and the amino acid sequence set forth in SEQ ID NO: 1 are aligned with each other, and a lysine is present at a position in the amino acid sequence while an amino acid other than lysine is present at a corresponding position in the amino acid sequence set forth in SEQ ID NO: 1, the lysine is substituted with an amino acid identical to the amino acid present at the corresponding position in the amino acid sequence set forth in SEQ ID NO: 1.

A fructosyl amino acid oxidase of the present invention may be one in which a residual activity exhibited by the fructosyl amino acid oxidase after being heat-treated while being immobilized on a support is higher than a residual activity exhibited by a fructosyl amino acid oxidase comprising an amino acid sequence identical to the foregoing amino acid sequence except that the amino acid substitutions as described in (7-1) and (7-2) are not incorporated.

Regarding the immobilization on the support, the fructosyl amino acid oxidase may be immobilized on the support as a result of crosslinking induced by an amine-reactive crosslinking agent. Furthermore, the heat treatment may be a treatment at a temperature of 48°C to 80°C.

In a fructosyl amino acid oxidase of the present invention, the position in (7-1) and (7-2) may be one or more selected from positions 56 to 99, 120 to 132, 155 to 171, and 353 to 404 of the amino acid sequence set forth in SEQ ID NO: 1; positions 100 to 119, 133 to 154, 241 to 288, and 435 to 440 of the amino acid sequence set forth in SEQ ID NO: 1; and positions 1 to 55, 172 to 240, 289 to 352, 405 to 434, and 441 to 446 of the amino acid sequence set forth in SEQ ID NO: 1.

A fructosyl amino acid oxidase of the present invention may be a fructosyl amino acid oxidase comprising an amino acid sequence that has a homology of 30% or greater to the amino acid sequence set forth in SEQ ID NO: 1 and in which, in an instance where the amino acid sequence and the amino acid sequence set forth in SEQ ID NO: 1 are aligned with each other, one or more of (a1) to (a6) described below are satisfied.

(a1) The amino acid at the position corresponding to position 61 of SEQ ID NO: 1 is an arginine.
(a2) The amino acid at the position corresponding to position 67 of SEQ ID NO: 1 is a glutamic acid.
(a3) The amino acid at the position corresponding to position 128 of SEQ ID NO: 1 is a lysine.
(a4) The amino acid at the position corresponding to position 157 of SEQ ID NO: 1 is a lysine.
(a5) The amino acid at the position corresponding to position 166 of SEQ ID NO: 1 is a lysine.
(a6) The amino acid at the position corresponding to position 391 of SEQ ID NO: 1 is a histidine.

A fructosyl amino acid oxidase of another aspect of the present invention may be a fructosyl amino acid oxidase comprising an amino acid sequence that has a homology of 30% or greater to the amino acid sequence set forth in SEQ ID NO: 1 and in which, in an instance where the amino acid sequence and the amino acid sequence set forth in SEQ ID NO: 1 are aligned with each other, one or more of (b1) to (b7) described below are satisfied.

(b1) The amino acid at the position corresponding to position 112 of SEQ ID NO: 1 is a lysine.
(b2) The amino acid at the position corresponding to position 115 of SEQ ID NO: 1 is a glutamic acid.
(b3) The amino acid at the position corresponding to position 137 of SEQ ID NO: 1 is a lysine.
(b4) The amino acid at the position corresponding to position 247 of SEQ ID NO: 1 is a lysine.
(b5) The amino acid at the position corresponding to position 254 of SEQ ID NO: 1 is a lysine.
(b6) The amino acid at the position corresponding to position 277 of SEQ ID NO: 1 is a lysine.
(b7) The amino acid at the position corresponding to position 438 of SEQ ID NO: 1 is a lysine.

A fructosyl amino acid oxidase of another aspect of the present invention may be a fructosyl amino acid oxidase comprising an amino acid sequence that has a homology of 30% or greater to the amino acid sequence set forth in SEQ ID NO: 1 and in which, in an instance where the amino acid sequence and the amino acid sequence set forth in SEQ ID NO: 1 are aligned with each other, one or more of (c1) to (c11) described below are satisfied.

(c1) The amino acid at the position corresponding to position 34 of SEQ ID NO: 1 is a lysine.
(c2) The amino acid at the position corresponding to position 37 of SEQ ID NO: 1 is a threonine.
(c3) The amino acid at the position corresponding to position 177 of SEQ ID NO: 1 is a lysine.
(c4) The amino acid at the position corresponding to position 198 of SEQ ID NO: 1 is a glutamic acid.
(c5) The amino acid at the position corresponding to position 297 of SEQ ID NO: 1 is a lysine.
(c6) The amino acid at the position corresponding to position 302 of SEQ ID NO: 1 is a valine.
(c7) The amino acid at the position corresponding to position 413 of SEQ ID NO: 1 is a lysine.
(c8) The amino acid at the position corresponding to position 424 of SEQ ID NO: 1 is a lysine.
(c9) The amino acid at the position corresponding to position 430 of SEQ ID NO: 1 is a lysine.
(c10) The amino acid at the position corresponding to position 432 of SEQ ID NO: 1 is a glycine.
(c11) The amino acid at the position corresponding to position 443 of SEQ ID NO: 1 is a lysine.

A fructosyl amino acid oxidase of another aspect of the present invention may be a fructosyl amino acid oxidase comprising an amino acid sequence that has a homology of 30% or greater to the amino acid sequence set forth in SEQ ID NO: 1 and in which, in an instance where the amino acid sequence and the amino acid sequence set forth in SEQ ID NO: 1 are aligned with each other, one or more of (a1) to (a6), (b1) to (b7), and (c1) to (c11) described below are satisfied.

(a1) The amino acid at the position corresponding to position 61 of SEQ ID NO: 1 is an arginine.
(a2) The amino acid at the position corresponding to position 67 of SEQ ID NO: 1 is a glutamic acid.

EP 4 471 135 A1

(a3) The amino acid at the position corresponding to position 128 of SEQ ID NO: 1 is a lysine.
(a4) The amino acid at the position corresponding to position 157 of SEQ ID NO: 1 is a lysine.
(a5) The amino acid at the position corresponding to position 166 of SEQ ID NO: 1 is a lysine.
(a6) The amino acid at the position corresponding to position 391 of SEQ ID NO: 1 is a histidine.
(b1) The amino acid at the position corresponding to position 112 of SEQ ID NO: 1 is a lysine.
(b2) The amino acid at the position corresponding to position 115 of SEQ ID NO: 1 is a glutamic acid.
(b3) The amino acid at the position corresponding to position 137 of SEQ ID NO: 1 is a lysine.
(b4) The amino acid at the position corresponding to position 247 of SEQ ID NO: 1 is a lysine.
(b5) The amino acid at the position corresponding to position 254 of SEQ ID NO: 1 is a lysine.
(b6) The amino acid at the position corresponding to position 277 of SEQ ID NO: 1 is a lysine.
(b7) The amino acid at the position corresponding to position 438 of SEQ ID NO: 1 is a lysine.
(c1) The amino acid at the position corresponding to position 34 of SEQ ID NO: 1 is a lysine.
(c2) The amino acid at the position corresponding to position 37 of SEQ ID NO: 1 is a threonine.
(c3) The amino acid at the position corresponding to position 177 of SEQ ID NO: 1 is a lysine.
(c4) The amino acid at the position corresponding to position 198 of SEQ ID NO: 1 is a glutamic acid.
(c5) The amino acid at the position corresponding to position 297 of SEQ ID NO: 1 is a lysine.
(c6) The amino acid at the position corresponding to position 302 of SEQ ID NO: 1 is a valine.
(c7) The amino acid at the position corresponding to position 413 of SEQ ID NO: 1 is a lysine.
(c8) The amino acid at the position corresponding to position 424 of SEQ ID NO: 1 is a lysine.
(c9) The amino acid at the position corresponding to position 430 of SEQ ID NO: 1 is a lysine.
(c10) The amino acid at the position corresponding to position 432 of SEQ ID NO: 1 is a glycine.
(c11) The amino acid at the position corresponding to position 443 of SEQ ID NO: 1 is a lysine.

A fructosyl amino acid of the present invention may have a specific reactivity with fructosyl lysine. Regarding the reactivity, the fructosyl amino acid oxidase may have a reactivity with fructosyl valine or a peptide containing fructosyl valine of 20 or less based on the reactivity with fructosyl lysine, which is taken as 100. Regarding the reactivity, the fructosyl amino acid oxidase may have a reactivity with fructosyl glycine of 20 or less based on the reactivity with fructosyl lysine, which is taken as 100.

Furthermore, the present invention provides a method for producing a fructosyl amino acid oxidase. The production method of the present invention is a method for producing a fructosyl amino acid oxidase including the step of substituting one or more amino acids in an amino acid sequence of a fructosyl amino acid oxidase with a lysine, the amino acid sequence having a homology of 30% or greater to an amino acid sequence set forth in SEQ ID NO: 1, the one or more amino acids being at one or more positions in the amino acid sequence that correspond to one or more selected from positions 309, 413, 424, 430, and 443 of the amino acid sequence set forth in SEQ ID NO: 1, in an instance where the amino acid sequence and the amino acid sequence set forth in SEQ ID NO: 1 are aligned with each other; and a thermal stability, which is exhibited while the fructosyl amino acid oxidase is immobilized on a support, of the fructosyl amino acid oxidase consisting of an amino acid sequence that has undergone the amino acid substitution is higher than a thermal stability, which is exhibited while the fructosyl amino acid oxidase is immobilized on a support, of the fructosyl amino acid oxidase consisting of the amino acid sequence that has not yet undergone the amino acid substitution.

Another method for producing a fructosyl amino acid oxidase is a method including the step of substituting amino acids in an amino acid sequence of a fructosyl amino acid oxidase with a lysine, the amino acid sequence having a homology of 30% or greater to an amino acid sequence set forth in SEQ ID NO: 1, the amino acids being amino acids described below in any one of (8-1) to (8-3); and a thermal stability, which is exhibited while the fructosyl amino acid oxidase is immobilized on a support, of the fructosyl amino acid oxidase consisting of an amino acid sequence that has undergone the amino acid substitution is higher than a thermal stability, which is exhibited while the fructosyl amino acid oxidase is immobilized on a support, of the fructosyl amino acid oxidase consisting of the amino acid sequence that has not yet undergone the amino acid substitution:

(8-1) amino acids at positions in the amino acid sequence that correspond to positions 64, 84, 87, 128, 157, 166, 167, 394, and 397 of the amino acid sequence set forth in SEQ ID NO: 1, in an instance where the amino acid sequence and the amino acid sequence set forth in SEQ ID NO: 1 are aligned with each other;
(8-2) amino acids at positions in the amino acid sequence that correspond to positions 112, 137, 152, 247, 254, 255, 267, 277, and 438 of the amino acid sequence set forth in SEQ ID NO: 1, in an instance where the amino acid sequence and the amino acid sequence set forth in SEQ ID NO: 1 are aligned with each other; and
(8-3) amino acids at positions in the amino acid sequence that correspond to positions 34, 177, 233, 234, 297, 309, 413, 424, 430, and 443 of the amino acid sequence set forth in SEQ ID NO: 1, in an instance where the amino acid sequence and the amino acid sequence set forth in SEQ ID NO: 1 are aligned with each other.

6

Another method for producing a fructosyl amino acid oxidase is a method including the step of performing amino acid substitution as described below in (9-1) and (9-2), on an amino acid sequence of a fructosyl amino acid oxidase, the amino acid sequence having a homology of 30% or greater to an amino acid sequence set forth in SEQ ID NO: 1; and a thermal stability, which is exhibited while the fructosyl amino acid oxidase is immobilized on a support, of the fructosyl amino acid oxidase consisting of an amino acid sequence that has undergone the amino acid substitution is higher than a thermal stability, which is exhibited while the fructosyl amino acid oxidase is immobilized on a support, of the fructosyl amino acid oxidase consisting of the amino acid sequence that has not yet undergone the amino acid substitution:

(9-1) in an instance where the amino acid sequence and the amino acid sequence set forth in SEQ ID NO: 1 are aligned with each other, and an amino acid other than lysine is present at a position in the amino acid sequence while a lysine is present at a corresponding position in the amino acid sequence set forth in SEQ ID NO: 1, the amino acid other than lysine is substituted with a lysine; and
(9-2) in an instance where the amino acid sequence and the amino acid sequence set forth in SEQ ID NO: 1 are aligned with each other, and a lysine is present at a position in the amino acid sequence while an amino acid other than lysine is present at a corresponding position in the amino acid sequence set forth in SEQ ID NO: 1, the lysine is substituted with an amino acid identical to the amino acid present at the corresponding position in the amino acid sequence set forth in SEQ ID NO: 1.

The thermal stability may be a thermal stability exhibited while the fructosyl amino acid oxidase is immobilized on the support as a result of crosslinking induced by an amine-reactive crosslinking agent. Furthermore, in a fructosyl amino acid oxidase produced by a production method of the present invention, a residual activity exhibited by the fructosyl amino acid oxidase after being treated at a temperature of 48°C while being immobilized on a support may be higher than a residual activity exhibited by the fructosyl amino acid oxidase after being treated under the same condition but without being immobilized on a support; and/or a residual activity exhibited by the fructosyl amino acid oxidase after being treated at a temperature of 65°C to 80°C for 20 minutes while being immobilized on a support may be 70% or greater. In the production methods of the present invention, the homology of the amino acid sequence may be 74% or greater.

Furthermore, the present invention provides a glycated protein sensor including a support and a fructosyl amino acid oxidase immobilized on the support, in which a residual activity exhibited by the fructosyl amino acid oxidase after being treated at a temperature of 65°C to 80°C for 20 minutes while being immobilized on a support is 70% or greater. The fructosyl amino acid oxidase may be immobilized on the support as a result of crosslinking induced by an amine-reactive crosslinking agent.

Furthermore, another aspect of the present invention provides a glycated protein sensor including a support and a fructosyl amino acid oxidase immobilized on the support, in which the fructosyl amino acid oxidase is any of the fructosyl amino acid oxidases described above. The fructosyl amino acid oxidase may be immobilized on the support as a result of crosslinking induced by an amine-reactive crosslinking agent.

Furthermore, the present invention provides a glycated protein sensor further including a hydrogen peroxide detector. In the glycated protein sensor, a support layer and the hydrogen peroxide detector may be layered on top of each other, and the support layer includes the support.

Furthermore, the present invention provides a method for measuring a glycated protein, the method including detecting a glycated protein by using a reaction of a fructosyl amino acid oxidase immobilized on a support.

ADVANTAGEOUS EFFECTS OF INVENTION

A fructosyl amino acid oxidase of the present invention has a specific reactivity with a particular glycated amino acid and/or a particular glycated peptide, among other glycated amino acids and/or glycated peptides. Using a fructosyl amino acid oxidase of the present invention enables accurate measurement of an amount of a particular glycated amino acid and/or a particular glycated peptide present in a test sample.

Furthermore, with the present invention, the thermal stability of a fructosyl amino acid oxidase immobilized on a support can be enhanced, and, therefore, a glycated protein sensor including the enzyme has improved long-term stability. A glycated protein sensor of the present invention is one in which a decrease in enzyme activity during storage is inhibited and, therefore, is advantageous in that the sensor can provide highly reliable measurement results over a long period of time.

BRIEF DESCRIPTION OF DRAWINGS

[Fig. 1] Fig. 1 shows the result of multiple alignment analysis of the amino acid sequences of fructosyl amino acid oxidases of Examples and Comparative Examples.

[Fig. 2] Fig. 2 shows the result of multiple alignment analysis of the amino acid sequences of fructosyl amino acid oxidases of Examples and Comparative Examples.

[Fig. 3] Fig. 3 is a schematic diagram of a glycated protein sensor 10, which is an embodiment of the present invention.

[Fig. 4] Fig. 4 is a schematic diagram of a glycated protein sensor 20, which is another embodiment of the present invention.

[Fig. 5] Fig. 5 shows the results of SDS-PAGE of fructosyl amino acid oxidases of Examples.

[Fig. 6] Fig. 6 shows the results of evaluating thermal stabilities exhibited by fructosyl amino acid oxidases of Examples in a liquid phase.

[Fig. 7] Fig. 7 shows the results of evaluating thermal stabilities exhibited by the fructosyl amino acid oxidases of the Examples while being immobilized on a support.

[Fig. 8] Fig. 8 shows the results of evaluating thermal stabilities exhibited by the fructosyl amino acid oxidases of Examples while being immobilized on a support.

[Fig. 9] Fig. 9 shows the results of evaluating thermal stabilities exhibited by the fructosyl amino acid oxidase of an Example and fructosyl amino acid oxidase variants thereof while being immobilized on a support.

DESCRIPTION OF EMBODIMENTS

Fructosyl amino acid oxidases of the present invention are novel fructosyl amino acid oxidases. The novel fructosyl amino acid oxidases of the present invention include fructosyl amino acid oxidases having high thermal stability. Furthermore, the present invention provides a method for producing a fructosyl amino acid oxidase modified to have enhanced thermal stability. Furthermore, the present invention provides a glycated protein sensor and a method for measuring a glycated protein. The sensor includes a support and a fructosyl amino acid oxidase immobilized on a support. The method detects a glycated protein by using a reaction of a fructosyl amino acid oxidase immobilized on a support. The present invention will be described in detail below with reference to embodiments.

A fructosyl amino acid oxidase is an enzyme that acts on an amino acid containing a glycated $\alpha$-amino group and/or a glycated $\varepsilon$-amino group or on a peptide containing such an amino acid and which produces hydrogen peroxide in the process of deglycating a glycated amino acid. A fructosyl amino acid oxidase (hereinafter also referred to as an "FAOD") is also referred to as an "amadoriase", a "ketoamine oxidase", or a "fructosyl amine oxidase". Fructosyl amino acid oxidases include a fructosyl peptide oxidase (hereinafter also referred to as an "FPOD" or an "FPOX"), which acts on glycated peptides.

Substrates of fructosyl amino acid oxidases are glycated amino acids and/or glycated peptides. Specific examples of the glycated amino acids include $\varepsilon$-fructosyl lysine (also referred to as "fructosyl lysine"), $\alpha$-fructosyl valine (also referred to as "fructosyl valine"), $\alpha$-fructosyl glycine (also referred to as "fructosyl glycine"), $\alpha$-fructosyl histiditine (also referred to as "fructosyl histidine"), $\alpha$-fructosyl leucine (also referred to as "fructosyl leucine"), and $\alpha$-fructosyl serine (also referred to as "fructosyl serine").

Specific examples of the glycated peptides include peptides composed of 2 to 10 amino acids, with one or more of the amino acids being glycated amino acids; the number of amino acids is preferably 2 to 6 and more preferably 2 or 3. One example is $\alpha$-fructosyl valyl histidine (also referred to as "fructosyl valyl histidine").

Fructosyl amino acid oxidases of the present invention have the following physicochemical properties. The fructosyl amino acid oxidases have an optimum pH range of pH 7 to 9, an operable pH range of pH 5 to 9, and an operable temperature range of 20 to 80°C and are soluble in a buffer solution. Specific examples of the buffer solution include Tris-hydrochloride buffers and phosphate buffered salines (PBS). A molecular weight on SDS-PAGE of the fructosyl amino acid oxidases of the present invention may be approximately 45 kDa to approximately 55 kDa and is preferably approximately 48 kDa to approximately 50 kDa.

A fructosyl amino acid oxidase according to an embodiment of the present invention is stable to heat while being immobilized on a support. The fructosyl amino acid oxidase that is stable to heat while being immobilized on a support is a fructosyl amino acid oxidase that exhibits a high residual activity after being heat-treated while being immobilized on a support. For example, a residual activity exhibited by the fructosyl amino acid oxidase after being treated at 65°C or greater is 70% or greater.

More specifically, the treatment temperature may be 48°C, 50°C, 55°C, 60°C, 65°C, 70°C, 75°C, 80°C, 85°C, 90°C, or 95°C. Furthermore, a treatment time is 10 to 30 minutes, preferably 15 to 25 minutes, and most preferably 20 minutes.

Another example of the fructosyl amino acid oxidase that is stable to heat while being immobilized on a support is one in which a residual activity exhibited by the fructosyl amino acid oxidase after being heat-treated while being immobilized on the support is higher than a residual activity exhibited by the fructosyl amino acid oxidase after being treated under the same condition but without being immobilized on a support. More specifically, the treatment temperature is 48°C, 50°C, 55°C, 60°C, 65°C, 70°C, 75°C, 80°C, 85°C, 90°C, or 95°C. Furthermore, a treatment time is 10 to 30 minutes, preferably 15 to 25 minutes, and most preferably 20 minutes.

The thermal stability of the fructosyl amino acid oxidase can be examined as follows: the support on which the fructosyl amino acid oxidase is immobilized is heated using a known means, such as an electric oven or a constant-temperature oven, and then the residual activity of the fructosyl amino acid oxidase is measured. As used herein, the term "residual activity" refers to a ratio of the enzyme activity exhibited after the heat treatment at various temperatures to the enzyme activity exhibited before the heat treatment.

Exemplary means for immobilizing enzymes, such as fructosyl amino acid oxidases, include covalent attachment methods, physical adsorption methods, ionic bonding methods, crosslinking methods, entrapment methods, and biochemical specific binding methods. An immobilizing method that does not cause inactivation of the enzyme may be selected in accordance with the enzyme that is used. Two or more immobilizing methods may be used in combination.

In some embodiments, the enzyme, alone or bound to a carrier, is immobilized onto a support by crosslinking induced by a crosslinking agent. In such instances, the support is a type of protein different from the enzyme, and the enzyme is immobilized onto the support by using a crosslinking agent, such as a glutaraldehyde or an isocyanate derivative, after mixing the enzyme with the support. Specific examples of the protein include albumins, such as bovine serum albumin (BSA), collagens, and gelatins. Alternatively, the support may be an enzyme identical to the enzyme that is immobilized. Such instances are sometimes called self-aggregation, in which the enzyme is immobilized onto an identical enzyme by crosslinking induced by a crosslinking agent.

The crosslinking agent to be used is a substance that undergoes inter- or intramolecular crosslinking, and specific examples thereof include glutaraldehydes, isocyanate derivatives, formaldehydes, glyoxals, malondialdehydes, and succinaldehydes. In particular, it is preferable to use an amine-reactive crosslinking agent as the crosslinking agent. The amine-reactive crosslinking agent is a crosslinking agent that crosslinks proteins by reacting with the amino group of the proteins. Specific examples of the amine-reactive crosslinking agent include glutaraldehydes, formaldehydes, N-hydroxy esters, amide esters, and imide esters.

The amine-reactive crosslinking agent reacts with the amino group present on a surface of the fructosyl amino acid oxidase, thereby causing crosslinking and thus immobilizing the fructosyl amino acid oxidase and the support. The fructosyl amino acid oxidase can be stable to heat while being immobilized on the support.

In other embodiments, the support may be a synthetic polymer, a resin, an inorganic material, a polysaccharide, a mineral, a clay, or the like. Specifically, any of the following means, for example, may be used: in instances where a fluororesin, an ion exchange resin, a polyvinyl alcohol resin, a water-curable resin, a photocurable resin, a solid polymer electrolyte, a polyion complex, a urethane resin, or the like is used as the support, the enzyme is immobilized onto the support by utilizing chemical interaction, such as hydrophobic interaction; in instances where charcoal, bone charcoal, a silica gel, glass, a zeolite, a Celite, an alumina, titanium oxide, a ceramic, a hydroxyapatite, or the like is used as the support, the enzyme is immobilized onto the support by utilizing physical interaction between the support and the enzyme: in instances where a semipermeable membrane made of nylon, cellophane, ethyl cellulose, acetyl cellulose, a polystyrene, or the like or a phospholipid membrane is used as the support, the enzyme is immobilized onto the support by being enclosed by the membrane; and in instances where a polyacrylamide gel, an agar, a gelatin, a carrageenan, a sodium alginate gel, a calcium alginate gel, a chitosan gel, or the like is used as the support, the enzyme is immobilized onto the gel.

The support may be beads. The beads may be carbon microparticles (carbon beads), silica ($SiO_2$) microparticles (silica beads), or beads made of a polysaccharide, such as chitin, chitosan, or alginic acid. The beads may include metal microparticles or a magnetizable substance and may be magnetic beads. The beads may have an average particle size of 10 nm or greater and 200 nm or less. The enzyme can be immobilized onto the beads by being crosslinked onto the beads.

A fructosyl amino acid oxidase of the present invention may specifically react with a particular glycated amino acid or a peptide containing the amino acid. In one embodiment, a fructosyl amino acid oxidase is highly specific for fructosyl lysine. The expression "being highly specific for fructosyl lysine" means that a reactivity with a different glycated amino acid or a peptide containing a different glycated amino acid is 20 or less based on the reactivity with fructosyl lysine or a peptide containing fructosyl lysine, which is taken as 100; the reactivity with a different glycated amino acid or a peptide containing a different glycated amino acid is preferably 10 or less, more preferably 5 or less, and even more preferably 1 or less. The different glycated amino acid is, for example, one or more selected from fructosyl valine, fructosyl glycine, fructosyl histidine, fructosyl leucine, and fructosyl serine.

Fructosyl amino acid oxidases can be classified into three groups according to their substrate specificity. Fructosyl amino acid oxidases belonging to the first group are highly specific for amino acids containing a glycated $\alpha$-amino group and/or for peptides containing such an amino acid. Fructosyl amino acid oxidases belonging to the second group are highly specific for amino acids containing a glycated $\varepsilon$-amino group and/or for peptides containing such an amino acid. Fructosyl amino acid oxidases belonging to the third group are highly specific for amino acids containing a glycated $\alpha$-amino group, for peptides containing such an amino acid, for amino acids containing a glycated $\varepsilon$-amino group, and/or for peptides containing such an amino acid. The fructosyl amino acid oxidases of the present invention may be fructosyl amino acid oxidases belonging to any of the groups.

In another embodiment, a fructosyl amino acid oxidase is highly specific for fructosyl valyl histidine. The expression "being highly specific for fructosyl valyl histidine" means that a reactivity with a different glycated amino acid or a different glycated peptide is 20 or less based on the reactivity with fructosyl valyl histidine, which is taken as 100; the reactivity with a different glycated amino acid or a different fructosy peptide is preferably 10 or less, more preferably 5 or less, and even more preferably 1 or less. The different glycated amino acid is, for example, one or more selected from fructosyl valine, fructosyl lysine, fructosyl glycine, fructosyl histidine, fructosyl leucine, and fructosyl serine, and the different fructosyl peptide is a peptide that contains a glycated amino acid and is different from fructosyl valyl histidine.

A fructosyl amino acid oxidase of the present invention may specifically react with a particular glycated amino acid or a peptide containing the amino acid. In one embodiment, a fructosyl amino acid oxidase is highly specific for fructosyl valine. The expression "being highly specific for fructosyl valine" means that a reactivity with a different glycated amino acid or a peptide containing a different glycated amino acid is 20 or less based on the reactivity with fructosyl valine or a peptide containing fructosyl valine, which is taken as 100; the reactivity with a different glycated amino acid or a peptide containing a different glycated amino acid is preferably 10 or less, more preferably 5 or less, and even more preferably 1 or less. The different glycated amino acid is, for example, one or more selected from fructosyl lysine, fructosyl glycine, fructosyl histidine, fructosyl leucine, and fructosyl serine.

A fructosyl amino acid oxidase of the present invention may be a recombinant protein expressed by transfecting a host cell with a nucleic acid encoding the fructosyl amino acid oxidase of the present invention. Examples of the host cells include Escherichia coli bacteria, fungi, yeasts, mammalian cells, and insect cells. Fructosyl amino acid oxidases that may be used include not only those that have been cloned from a microorganism, an animal, a plant, or the like but also those into which a mutation has been artificially introduced and those that have been modified or designed.

The inventors discovered novel fructosyl amino acid oxidases among genes of unknown function. Among them, a fructosyl amino acid oxidase having a significantly high thermal stability, which was a thermal stability exhibited while the fructosyl amino acid oxidase was immobilized on a support, was discovered. Accordingly, based on the amino acid sequence of the fructosyl amino acid oxidase, the inventors created other fructosyl amino acid oxidases as variants and thus succeeded in enhancing their thermal stability. The amino acid sequences of the novel fructosyl amino acid oxidases discovered by the inventors are shown in Table 1.

[Table 1]

| SEQ ID NO: | Origin (Accession Number) | Amino Acid Sequence | Homology to SEQ ID NO: 1 (%) |
|---|---|---|---|
| 1 | *Aspergillus pseudotamarii* (XP_031920077) | MASSNLTLTSSILIVGAGTWGCSTALHLARRGYKNVTVLDPHPVPSPIAAGNDINKIMEH REVKASETDPWSIAFSTCTRAALKGWKTDPVFQPYFHETGAIVSGHTPSLIKHIEEHEID SSDAEFVKLNTAEDFRKTMPPGILTGNFPGWKGWLNKTGAGWIHAKKAMFSAYTEAKRLG VTFITGSPEGDVVSLVYENGDVAGARTADGTIHRAEHTILSAGAGSDRLLDFKKQLRPTA WTLCHIKMTPEEAKKYRNLPVLFNVAKGFFMEPDEDKHELKICDEHPGYCNFIPDPKHGG EVRSIPFAKHQIPLEAEARARDFLRDTMPHLADRPLSFARICWDADTVDRAFLIDRHPEY RSLLLAVGGSGNGAMQMSTIGGFITDALEGHLQKELKHALRWRPEIATERDWKDTQNRFG GPNKVMDFQKVGENEWTKIGDDKCRL | - |

(continued)

| SEQ ID NO: | Origin (Accession Number) | Amino Acid Sequence | Homology to SEQ ID NO: 1 (%) |
|---|---|---|---|
| 2 | *Penicillium rubens* (XP_002568014) | MATLELNHTTSILIVGAGTWGCSTALHLARRGYRNVKVLDPHPVPSPIAAGNDINKIMEH KELKSPKPDARSIAFATCTRAALKGWKTDPVFQPYFHETGFIVSGHTPALIEHIKTEEID PSEGDWVALESAEDFRQTMPPGVLTGDFPGWKGWISRVGAGWIHARKAMVSAYNEAQRLG VDFVTGSPQGNVESLIYKDADVVGARTADGLSHYADRTILAAGAGSDRLLDFKKQLRPTA WTLSHIRMTPEEADKYRNLPVLFNVAKGFFMEPDEEKHELKICDEHPGYCNFVVDPDHQG EKRSIPFAKEQIPLEAEARARDFLRDTMPHLADRPFSFARICWDADTVDRAFLIDRHPEY ASLVVAVGGSGNGAMQMPTIGGFISDTLEGKLQKELKDVVRWRPETAIHRDWKSTQNRFG GPDRLMDFQDVKDDEWTRIGEDMASRL | 79 |
| 3 | *Penicillium flavigenum* (OQE32666) | MATLELNHASSILIVGAGTWGCSTALHLARRGYKNVKVLDPHPVPSPIAAGNDINKIMEH KELKSPKPDARSIAFATCTRAALKGWKTDPVFQPYFHETGFIVSGHTPALIEHIKTEDID PSEGDWAALESAEDFRQTMPPGVLTGDFPGWKGWISRVGTGWIHARKAMVSAYNEAQRLG VSFVTGSPEGNVESLIYKDGDVVGARTADGLSHYADRTILAAGAGSDRLLDFKKQLRPTA WTLSHIRMTPEEAEKYRNLPVLFNVAKGFFMEPDEEKHELKICDEHPGYCNFVVDPDHQG EKRSIPFAKEQIPLEAEARARDFLRDTMPHLADRPFSFARICWDADTVDRAFLIDRHPEY ASLVVAVGGSGNGAMQMPTIGGFISDVLEGKLQKELKDVVRWRPEIAIRRDWKSTQNRFG GPDRLMDFQDVKDDEWTSIGEEMASRL | 75 |
| 4 | *Monascus purpureus* (TQB73262) | MASELSTSSSILIVGAGTWGCSTALHLARRGYKHVTVLDPYPVPSPIAAGNDVNKIMEHK EVAGIIAFATCTRAALKGWQTDPVFKPYFHETGFIQAGHTPALIEHIRQNDLYPSESDFV KLETAEDFRKTMPPGVLTGDFPGWKGWLMKKNAGWIHARKAMFSAFHEAKRLGVTFITGS PQGKVVSLVYGQDDVTGARTEDGKVHSADRVIVSAGAGTDRILDMENQLRPTAWTLCHIK MTEEEIKSQRYRNLPVLFNIAKGFFMEPDEDKGELKMCDEHPGYVNWVSDPNRPGEKRSI PFAKEQIPREAERRAREFLKDTMPHLADRPLVFARICWDADTPDRFFLIDYHPRYPSLLL ACGGSGNGAMQMPSIGGFIADALEGRLQEELKDVCRWRPETAVGRDWKSTQGRFGGPDKV MDFQEVGENEWTRVGKESLL | 79 |

(continued)

| SEQ ID NO: | Origin (Accession Number) | Amino Acid Sequence | Homology to SEQ ID NO: 1 (%) |
|---|---|---|---|
| 5 | *Coniochaeta pulveracea* (RKU49498) | MAGGLGTQSRILILGGGTWGCSTALHLARRGYKNVTVLDAHPIPSPISAGNDVNKIVEQG<br><br>CFTDGDDEAAVAQTLLHAANEGWQKDPVFQPYYHDTGYIVAGSSPKAIERLVDREVRHQA<br><br>SSFRKLTTAEDFKGTMPTGVLTGDFPGWEGYYKPSGAGWVHARKALVSAYNEAKRLGVNF<br><br>ITGSPEGKVKSLLLEDGDVRGAITADGVEHRADRTILAMGANAPQLLDFENQLRPTAWTL<br><br>AHIPMTEEETRLYKNLPVLFNIEKGFFMEPDEDKHELKLCDEHPGYCNWVDQPGSALPQS<br><br>VPVAKHEVPASAERRMRDFLREIMPHLADRPFTFARMCWCADTRNRAFLITYHPKHPSLV<br><br>VASGDSGHGFMHIPSIGGFIVDCMENKLQPNFAKSWRWRPETTRDFWGRDVLDRFGAGNK<br><br>MLDVNETREEGWTNVNATPLTVA | 58 |

The fructosyl amino acid oxidase consisting of the amino acid sequence set forth in SEQ ID NO: 1 is derived from Aspergillus pseudotamarii, which belongs to the genus Aspergillus. So far, the protein has been assumed to be a type of FAD-dependent oxidoreductase (Accession Number XP_031920077), based on a homology of the sequence, but it has not been known that the protein could function as a fructosyl amino acid oxidase. The present inventors discovered that the fructosyl amino acid oxidase consisting of the amino acid sequence set forth in SEQ ID NO: 1 exhibits a significantly high thermal stability while being immobilized on a support and that the fructosyl amino acid oxidase has a very high substrate specificity for a particular glycated amino acid and/or glycated peptide.

The fructosyl amino acid oxidase consisting of the amino acid sequence set forth in SEQ ID NO: 2 is derived from Penicillium rubens, which belongs to the genus Penicillium. So far, the protein has been known as a hypothetical protein (Accession Number XP_002568014), but it has not been known that the protein could function as a fructosyl amino acid oxidase. The present inventors discovered that the fructosyl amino acid oxidase consisting of the amino acid sequence set forth in SEQ ID NO: 2 has a very high substrate specificity for a particular glycated amino acid and/or glycated peptide.

The fructosyl amino acid oxidase consisting of the amino acid sequence set forth in SEQ ID NO: 3 is derived from Penicillium flavigenum, which belongs to the genus Penicillium. So far, the protein has been known as a hypothetical protein (Accession Number OQE32666), but it has not been known that the protein could function as a fructosyl amino acid oxidase. The present inventors discovered that the fructosyl amino acid oxidase consisting of the amino acid sequence set forth in SEQ ID NO: 3 has a very high substrate specificity for a particular glycated amino acid and/or glycated peptide.

The fructosyl amino acid oxidase consisting of the amino acid sequence set forth in SEQ ID NO: 4 is derived from Monascus purpureus, which belongs to the genus Monascus. So far, the protein has been known as a hypothetical protein (Accession Number TQB73262), but it has not been known that the protein could function as a fructosyl amino acid oxidase. The present inventors discovered that the fructosyl amino acid oxidase consisting of the amino acid sequence set forth in SEQ ID NO: 4 has a very high substrate specificity for a particular glycated amino acid and/or glycated peptide.

The fructosyl amino acid oxidase consisting of the amino acid sequence set forth in SEQ ID NO: 5 is derived from Coniochaeta pulveracea, which belongs to the genus Coniochaeta. So far, the protein has been known as a hypothetical protein (Accession Number RKU49498), but it has not been known that the protein could function as a fructosyl amino acid oxidase. The present inventors discovered that the fructosyl amino acid oxidase consisting of the amino acid sequence set forth in SEQ ID NO: 5 has a very high substrate specificity for a particular glycated amino acid and/or glycated peptide.

The present invention may also use a fructosyl amino acid oxidase that has an amino acid sequence having a high homology to the amino acid sequence of any of SEQ ID NOs: 1 to 5 and which has physicochemical properties such as high thermal stability exhibited while the fructosyl amino acid oxidase is immobilized on a support and high substrate specificity for a particular glycated amino acid or glycated peptide. Specifically, the amino acid sequence of the fructosyl amino acid oxidase may include an amino acid sequence having a homology, to the amino acid sequence of SEQ ID NO: 1, of 30% or greater, 35% or greater, 40% or greater, 45% or greater, 50% or greater, 51% or greater, 52% or greater, 53% or greater, 54% or greater, 55% or greater, 56% or greater, 57% or greater, 58% or greater, 59% or greater, 60% or greater, 61% or greater, 62% or greater, 63% or greater, 64% or greater, 65% or greater, 66% or greater, 67% or greater, 68% or greater, 69% or greater, 70% or greater, 71% or greater, 72% or greater, 73% or greater, 74% or greater, 75% or greater, 76% or greater, 77% or greater, 78% or greater, 79% or greater, or 80% or greater. These homology values have been rounded to the nearest whole number.

In another embodiment of the present invention, a fructosyl amino acid oxidase included in a glycated protein sensor may be a fructosyl amino acid oxidase consisting of the amino acid sequence of SEQ ID NO: 1 or an amino acid sequence other than the amino acid sequence of SEQ ID NO: 1. The fructosyl amino acid oxidase consisting of an amino acid sequence other than the amino acid sequence of SEQ ID NO: 1 may be a variant of a fructosyl amino acid oxidase, which may be a variant to which physicochemical properties have been imparted, and examples of the physicochemical properties may include high thermal stability exhibited while the fructosyl amino acid oxidase is immobilized on a support and high substrate specificity for a particular glycated amino acid or glycated peptide.

As will be described later, the fructosyl amino acid oxidase consisting of the amino acid sequence set forth in SEQ ID NO: 1 was found to have excellent physicochemical properties, including very high stability to heat exhibited while the fructosyl amino acid oxidase is immobilized on a support; for example, a residual activity exhibited by the fructosyl amino acid oxidase after being treated at a temperature of 65°C to 80°C for 20 minutes while being immobilized on a support is 70% or greater, and/or a residual activity exhibited by the fructosyl amino acid oxidase after being heat-treated while being immobilized on a support is higher than a residual activity exhibited by the fructosyl amino acid oxidase after being treated under the same conditions but without being immobilized on a support.

Furthermore, the inventors thoroughly investigated the amino acid sequence set forth in SEQ ID NO: 1 and found that lysines at positions 247 and/or 277 and lysines at positions 430 and/or 443 in the amino acid sequence set forth in SEQ ID NO: 1 play an important role for the thermal stability of the enzyme.

Specifically, one embodiment of the present invention may be a fructosyl amino acid oxidase consisting of an amino acid sequence that has a high homology to the amino acid sequence set forth in SEQ ID NO: 1 and which satisfies the conditions described below in (1) and (2), or the embodiment may be a fructosyl amino acid oxidase including the amino acid sequence. (1) In an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines. (2) In an instance where the amino acid sequence and the amino acid sequence set forth in SEQ ID NO: 1 are aligned with each other, amino acids at positions in the amino acid sequence that correspond to positions 430 and/or 443 of the amino acid sequence set forth in SEQ ID NO: 1 are lysines.

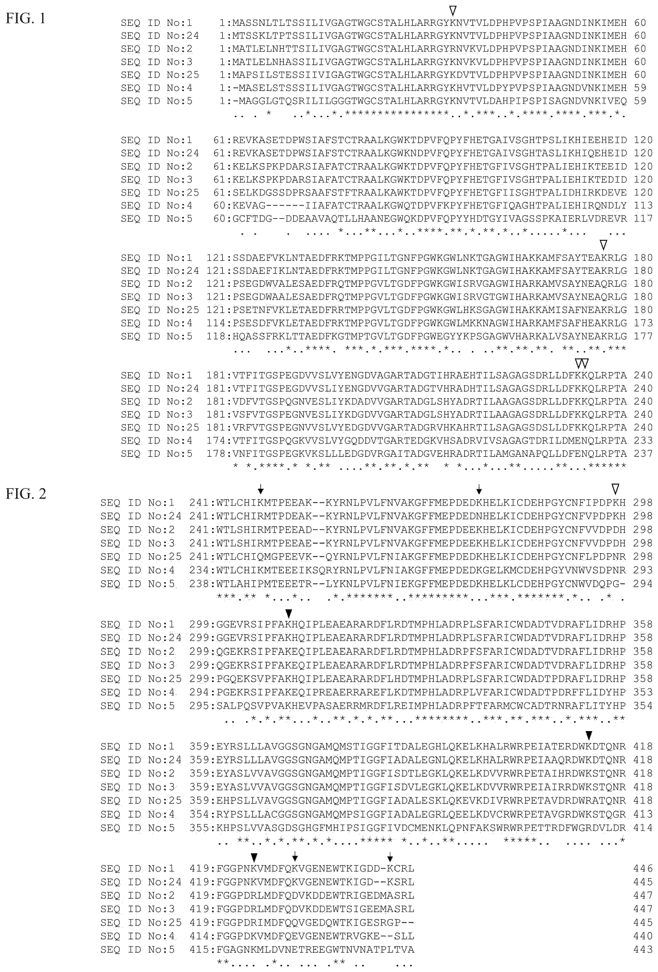

Figs. 1 and 2 show the results of multiple alignment analysis performed on SEQ ID NOs: 1 to 5, described above, and SEQ ID NOs: 24 and 25, which are amino acid sequences of known fructosyl amino acid oxidases. SEQ ID NO: 24 is the sequence of a fructosyl amino acid oxidase (Accession Number BAD54824) derived from Aspergillus oryzae, and SEQ ID NO: 25 is the sequence of a fructosyl amino acid oxidase (Accession Number AAB88209) derived from Aspergillus fumigatus. In the figures, the amino acids at positions 247, 277, 430, and 443 of the amino acid sequence set forth in SEQ ID NO: 1 are indicated by arrows. Furthermore, Table 2 shows the amino acids of SEQ ID NOs: 2 to 5, 24 and 25 that correspond to lysines at positions 247, 277, 430, and 443 of the amino acid sequence set forth in SEQ ID NO: 1. As shown in Table 2, the amino acid sequences that satisfy (1) and (2), described above, are distinguished from SEQ ID NOs: 24 and 25, which are amino acid sequences of known fructosyl amino acid oxidases.

Another embodiment of the present invention may be a fructosyl amino acid oxidase consisting of an amino acid sequence that has a high homology to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 247, 277, 430, and 443 of the amino acid sequence set forth in SEQ ID NO: 1 are lysines, or the embodiment may be a fructosyl amino acid oxidase comprising the amino acid sequence.

[Table 2]

| Position | SEQ ID NO: | | | | | | |
|---|---|---|---|---|---|---|---|
| | 1 | 2 | 3 | 4 | 5 | 24 | 25 |
| 247 | K | R | R | K | P | R | Q |
| 277 | K | K | K | K | K | N | K |
| 430 | K | D | D | E | E | K | Q |
| 443 | K | A | A | - | L | K | G |

Furthermore, it was also found that in addition to the amino acids described above, lysines at positions 309, 413, and 424 in the amino acid sequence of SEQ ID NO: 1 also play an important role for the thermal stability of the enzyme. Specifically, one embodiment of the present invention may be a fructosyl amino acid oxidase consisting of an amino acid sequence that has a high homology to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 309, 413, and/or 424 of the amino acid sequence set forth in SEQ ID NO: 1 are lysines, or the embodiment may be a fructosyl amino acid oxidase comprising the amino acid sequence. In Fig. 2, the amino acids at positions 309, 413, and 424 of the amino acid sequence set forth in SEQ ID NO: 1 are indicated by arrowheads.

Another embodiment of the present invention may be a fructosyl amino acid oxidase consisting of an amino acid sequence that has a high homology to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 309 and 413 and/or to position 443 of the amino acid sequence set forth in SEQ ID NO: 1 are lysines, or the embodiment may be a fructosyl amino acid oxidase comprising the amino acid sequence.

Furthermore, it was also found that in addition to the amino acids described above, lysines at positions 34, 177, 233, 234, and 297 in the amino acid sequence of SEQ ID NO: 1 also play an important role for the thermal stability of the enzyme. Specifically, one embodiment of the present invention may be a fructosyl amino acid oxidase consisting of an amino acid sequence that has a high homology to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 34, 177, 233, 234, and/or 297 of the amino acid sequence set forth in SEQ ID NO: 1 are lysines, or the embodiment may be a fructosyl amino acid oxidase comprising the amino acid sequence. In Figs. 1 and 2, the amino acids at positions 34, 177, 233, 234, and 297 of the amino acid sequence set forth in SEQ ID NO: 1 are indicated by white arrowheads.

Another embodiment of the present invention may be a fructosyl amino acid oxidase consisting of an amino acid sequence that has a high homology to the amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 34, 177, 233, 234, and 297 of the amino acid sequence set forth in SEQ ID NO: 1 are lysines, or the embodiment may be a fructosyl amino acid oxidase comprising the amino acid sequence.

Furthermore, it was also found that in addition to the amino acids described above, lysines at positions 64, 84, 87, 128, 157, 166, 167, 394, and 397 and lysines at positions 112, 137, 152, 247, 254, 255, 267, 277, and 438, in the amino acid sequence of SEQ ID NO: 1 also play an important role for the thermal stability of the enzyme.

Specifically, one embodiment of the present invention may be a fructosyl amino acid oxidase consisting of an amino acid sequence that has a high homology to the amino acid sequence set forth in SEQ ID NO: 1 and which satisfies the conditions described below in (3-1) and/or (4-1), or the embodiment may be a fructosyl amino acid oxidase comprising the amino acid sequence. (3-1) In an instance where the amino acid sequence and the amino acid sequence set forth in SEQ ID NO: 1 are aligned with each other, amino acids at one or more positions in the amino acid sequence that correspond to one or more selected from positions 64, 84, 87, 128, 157, 166, 167, 394, and 397 of the amino acid sequence set forth in SEQ ID NO: 1 are lysines. (4-1) In an instance where the amino acid sequence and the amino acid sequence set forth in SEQ ID NO: 1 are aligned with each other, amino acids at one or more positions in the amino acid sequence that correspond to one or more selected from positions 112, 137, 152, 247, 254, 255, 267, 277, and 438 of the amino acid sequence set forth in SEQ ID NO: 1 are lysines.

Another embodiment of the present invention may be a fructosyl amino acid oxidase consisting of an amino acid sequence that has a high homology to the amino acid sequence set forth in SEQ ID NO: 1 and which satisfies the conditions described below in (3-2) and/or (4-2), or the embodiment may be a fructosyl amino acid oxidase comprising the amino acid sequence. (3-2) In an instance where the amino acid sequence and the amino acid sequence set forth in SEQ ID NO: 1 are aligned with each other, amino acids at positions in the amino acid sequence that correspond to positions 64, 84, 87, 128, 157, 166, 167, 394, and 397 of the amino acid sequence set forth in SEQ ID NO: 1 are lysines. (4-2) In an instance where the amino acid sequence and the amino acid sequence set forth in SEQ ID NO: 1 are aligned with each other, amino acids at positions in the amino acid sequence that correspond to positions 112, 137, 152, 247, 254, 255, 267, 277, and 438 of the amino acid sequence set forth in SEQ ID NO: 1 are lysines.

Furthermore, the present invention provides a method for producing a fructosyl amino acid oxidase that includes a modified amino acid sequence. The method of the present invention for producing a fructosyl amino acid oxidase includes the step of substituting an amino acid in the amino acid sequence of a fructosyl amino acid oxidase. More specifically, the step may be a step of substituting one or more amino acids in an amino acid sequence of a fructosyl amino acid oxidase with a lysine, where the amino acid sequence (hereinafter referred to as a "pre-modification amino acid sequence") has a high homology to the amino acid sequence set forth in SEQ ID NO: 1 and is one in which, in an instance where the pre-modification amino acid sequence and the amino acid sequence set forth in SEQ ID NO: 1 are aligned with each other, the one or more amino acids in the pre-modification amino acid sequence are those at positions corresponding to positions 34, 64, 84, 87, 112, 177, 128, 137, 152, 157, 166, 167, 233, 234, 247, 254, 255, 267, 277, 297, 309, 394, 397, 413, 424, 430, 438, and 443 of the amino acid sequence set forth in SEQ ID NO: 1.

The above-mentioned amino acids in the amino acid sequence set forth in SEQ ID NO: 1 have been found to play an important role for the thermal stability of the enzyme. Accordingly, the fructosyl amino acid oxidase modified by the production method of the present invention has higher thermal stability than the pre-modification fructosyl amino acid oxidase. More preferably, the fructosyl amino acid oxidase modified by the production method of the present invention is one in which a thermal stability exhibited while the fructosyl amino acid oxidase is immobilized on a support is higher than that of the pre-modification fructosyl amino acid oxidase.

The present inventors discovered that the physicochemical properties of a fructosyl amino acid oxidase can be improved by dividing SEQ ID NO: 1 into three regions (a), (b), and (c) based on the steric structure of the fructosyl amino acid oxidase and modifying amino acids for each of the regions. Region (a) is a region composed of amino acids corresponding to positions 56 to 99, 120 to 132, 155 to 171, and 353 to 404 of SEQ ID NO: 1. Region (b) is a region composed of amino acids corresponding to positions 100 to 119, 133 to 154, 241 to 288, and 435 to 440 of SEQ ID NO: 1. Region (c) is a region composed of amino acids corresponding to positions 1 to 55, 172 to 240, 289 to 352, 405 to 434, and 441 to 446 of SEQ ID NO: 1.

Another embodiment of the method of the present invention for producing a fructosyl amino acid oxidase includes the step of modifying amino acids in a fructosyl amino acid oxidase that consists of an amino acid sequence having a high homology to that of SEQ ID NO: 1, the modifying being performed for each of the regions in a manner that satisfies conditions (1) and (2) described below. With this production method, it is possible to obtain an enzyme having higher thermal stability than the pre-modification enzyme.

(1) In an instance where the pre-modification amino acid sequence and the amino acid sequence set forth in SEQ ID NO: 1 are aligned with each other, and an amino acid other than lysine is present at a position in the pre-modification amino acid sequence while a lysine is present at a corresponding position in the amino acid sequence of SEQ ID NO: 1, the amino acid other than lysine is substituted with a lysine.

(2) In an instance where the pre-modification amino acid sequence and the amino acid sequence set forth in SEQ ID NO: 1 are aligned with each other, and a lysine is present at a position in the pre-modification amino acid sequence while an amino acid other than lysine is present at a corresponding position in the amino acid sequence of SEQ ID NO: 1, the lysine is substituted with an amino acid identical to the amino acid other than lysine, that is, an amino acid identical to the amino acid at the corresponding position in the amino acid sequence set forth in SEQ ID NO: 1.

Note that the alignment of the amino acid sequences can be carried out by a program, such as Blast, Clustal W, or Clustal Omega. The alignment can provide information, such as homology, identity, or similarity between multiple amino acid sequences and corresponding amino acid positions.

An example of a variant of a fructosyl amino acid oxidase that can be provided by the present invention is described. The fructosyl amino acid oxidase is a fructosyl amino acid oxidase modified such that, in an instance where the amino acid sequence of the fructosyl amino acid oxidase and the amino acid sequence set forth in SEQ ID NO: 1 are aligned with each other, one or more selected from the group consisting of (a1) to (a6), (b1) to (b7), and (c1) to (c11) described below are satisfied. A thermal stability exhibited by the fructosyl amino acid oxidase while being immobilized on a support is improved compared to that of the pre-modification fructosyl amino acid oxidase.

(a1) The amino acid at the position corresponding to position 61 of SEQ ID NO: 1 is an arginine.
(a2) The amino acid at the position corresponding to position 67 of SEQ ID NO: 1 is a glutamic acid.
(a3) The amino acid at the position corresponding to position 128 of SEQ ID NO: 1 is a lysine.
(a4) The amino acid at the position corresponding to position 157 of SEQ ID NO: 1 is a lysine.
(a5) The amino acid at the position corresponding to position 166 of SEQ ID NO: 1 is a lysine.
(a6) The amino acid at the position corresponding to position 391 of SEQ ID NO: 1 is a histidine.
(b1) The amino acid at the position corresponding to position 112 of SEQ ID NO: 1 is a lysine.
(b2) The amino acid at the position corresponding to position 115 of SEQ ID NO: 1 is a glutamic acid.
(b3) The amino acid at the position corresponding to position 137 of SEQ ID NO: 1 is a lysine.
(b4) The amino acid at the position corresponding to position 247 of SEQ ID NO: 1 is a lysine.
(b5) The amino acid at the position corresponding to position 254 of SEQ ID NO: 1 is a lysine.
(b6) The amino acid at the position corresponding to position 277 of SEQ ID NO: 1 is a lysine.
(b7) The amino acid at the position corresponding to position 438 of SEQ ID NO: 1 is a lysine.
(c1) The amino acid at the position corresponding to position 34 of SEQ ID NO: 1 is a lysine.
(c2) The amino acid at the position corresponding to position 37 of SEQ ID NO: 1 is a threonine.
(c3) The amino acid at the position corresponding to position 177 of SEQ ID NO: 1 is a lysine.

(c4) The amino acid at the position corresponding to position 198 of SEQ ID NO: 1 is a glutamic acid.
(c5) The amino acid at the position corresponding to position 297 of SEQ ID NO: 1 is a lysine.
(c6) The amino acid at the position corresponding to position 302 of SEQ ID NO: 1 is a valine.
(c7) The amino acid at the position corresponding to position 413 of SEQ ID NO: 1 is a lysine.
(c8) The amino acid at the position corresponding to position 424 of SEQ ID NO: 1 is a lysine.
(c9) The amino acid at the position corresponding to position 430 of SEQ ID NO: 1 is a lysine.
(c10) The amino acid at the position corresponding to position 432 of SEQ ID NO: 1 is a glycine.
(c11) The amino acid at the position corresponding to position 443 of SEQ ID NO: 1 is a lysine.

Specific examples of the amino acid sequences of modified fructosyl amino acid oxidases are described. The amino acid sequence set forth in SEQ ID NO: 6 is an amino acid sequence modified from the amino acid sequence set forth in SEQ ID NO: 2. The amino acid sequence set forth in SEQ ID NO: 2 and the amino acid sequence set forth in SEQ ID NO: 1 were aligned with each other, and amino acids were substituted as described below to give the amino acid sequence set forth in SEQ ID NO: 6.

(a1) A lysine at position 61 of SEQ ID NO: 2 (corresponding to position 61 of SEQ ID NO: 1) was substituted with an arginine.
(a2) A lysine at position 67 of SEQ ID NO: 2 (corresponding to position 67 of SEQ ID NO: 1) was substituted with a glutamic acid.
(a3) An alanine at position 128 of SEQ ID NO: 2 (corresponding to position 128 of SEQ ID NO: 1) was substituted with a lysine.
(a4) An arginine at position 157 of SEQ ID NO: 2 (corresponding to position 157 of SEQ ID NO: 1) was substituted with a lysine.
(a5) An arginine at position 166 of SEQ ID NO: 2 (corresponding to position 166 of SEQ ID NO: 1) was substituted with a lysine.
(a6) A lysine at position 391 of SEQ ID NO: 2 (corresponding to position 391 of SEQ ID NO: 1) was substituted with histidine.

The amino acid sequence set forth in SEQ ID NO: 7 is an amino acid sequence modified from the amino acid sequence set forth in SEQ ID NO: 3. The amino acid sequence set forth in SEQ ID NO: 3 and the amino acid sequence set forth in SEQ ID NO: 1 were aligned with each other, and amino acids were substituted as described below to give the amino acid sequence set forth in SEQ ID NO: 7.

(a1) A lysine at position 61 of SEQ ID NO: 3 (corresponding to position 61 of SEQ ID NO: 1) was substituted with an arginine.
(a2) A lysine at position 67 of SEQ ID NO: 3 (corresponding to position 67 of SEQ ID NO: 1) was substituted with a glutamic acid.
(a3) An alanine at position 128 of SEQ ID NO: 3 (corresponding to position 128 of SEQ ID NO: 1) was substituted with a lysine.
(a4) An arginine at position 157 of SEQ ID NO: 3 (corresponding to position 157 of SEQ ID NO: 1) was substituted with a lysine.
(a5) An arginine at position 166 of SEQ ID NO: 3 (corresponding to position 166 of SEQ ID NO: 1) was substituted with a lysine.
(a6) A lysine at position 391 of SEQ ID NO: 3 (corresponding to position 391 of SEQ ID NO: 1) was substituted with a histidine.

The amino acid sequence set forth in SEQ ID NO: 8 is an amino acid sequence modified from the amino acid sequence set forth in SEQ ID NO: 4. The amino acid sequence set forth in SEQ ID NO: 4 and the amino acid sequence set forth in SEQ ID NO: 1 were aligned with each other, and amino acids were substituted as described below to give the amino acid sequence set forth in SEQ ID NO: 8.

(a1) A lysine at position 60 of SEQ ID NO: 4 (corresponding to position 61 of SEQ ID NO: 1) was substituted with an arginine.
(a5) An arginine at position 159 of SEQ ID NO: 4 (corresponding to position 166 of SEQ ID NO: 1) was substituted with a lysine.

The amino acid sequence set forth in SEQ ID NO: 9 is an amino acid sequence modified from the amino acid sequence set forth in SEQ ID NO: 5. The amino acid sequence set forth in SEQ ID NO: 5 and the amino acid sequence set

16

forth in SEQ ID NO: 1 were aligned with each other, and amino acids were substituted as described below to give the amino acid sequence set forth in SEQ ID NO: 9.

(a4) A proline at position 154 of SEQ ID NO: 5 (corresponding to position 157 of SEQ ID NO: 1) was substituted with a lysine.
(a5) An arginine at position 163 of SEQ ID NO: 5 (corresponding to position 166 of SEQ ID NO: 1) was substituted with a lysine.
(a6) A lysine at position 387 of SEQ ID NO: 5 (corresponding to position 391 of SEQ ID NO: 1) was substituted with a histidine.

The amino acid sequence set forth in SEQ ID NO: 10 is an amino acid sequence modified from the amino acid sequence set forth in SEQ ID NO: 2. The amino acid sequence set forth in SEQ ID NO: 2 and the amino acid sequence set forth in SEQ ID NO: 1 were aligned with each other, and amino acids were substituted as described below to give the amino acid sequence set forth in SEQ ID NO: 10.

(b 1) A glutamic acid at position 112 of SEQ ID NO: 2 (corresponding to position 112 of SEQ ID NO: 1) was substituted with a lysine.
(b2) A lysine at position 115 of SEQ ID NO: 2 (corresponding to position 115 of SEQ ID NO: 1) was substituted with a glutamic acid.
(b3) A glutamine at position 137 of SEQ ID NO: 2 (corresponding to position 137 of SEQ ID NO: 1) was substituted with a lysine.
(b4) An arginine at position 247 of SEQ ID NO: 2 (corresponding to position 247 of SEQ ID NO: 1) was substituted with a lysine.
(b5) An aspartic acid at position 254 of SEQ ID NO: 2 (corresponding to position 254 of SEQ ID NO: 1) was substituted with a lysine.
(b7) An arginine at position 438 of SEQ ID NO: 2 (corresponding to position 438 of SEQ ID NO: 1) was substituted with a lysine.

The amino acid sequence set forth in SEQ ID NO: 11 is an amino acid sequence modified from the amino acid sequence set forth in SEQ ID NO: 3. The amino acid sequence set forth in SEQ ID NO: 3 and the amino acid sequence set forth in SEQ ID NO: 1 were aligned with each other, and amino acids were substituted as described below to give the amino acid sequence set forth in SEQ ID NO: 11.

(b 1) A glutamic acid at position 112 of SEQ ID NO: 3 (corresponding to position 112 of SEQ ID NO: 1) was substituted with a lysine.
(b2) A lysine at position 115 of SEQ ID NO: 3 (corresponding to position 115 of SEQ ID NO: 1) was substituted with a glutamic acid.
(b3) A glutamine at position 137 of SEQ ID NO: 3 (corresponding to position 137 of SEQ ID NO: 1) was substituted with a lysine.
(b4) An arginine at position 247 of SEQ ID NO: 3 (corresponding to position 247 of SEQ ID NO: 1) was substituted with a lysine.
(b5) A glutamic acid at position 254 of SEQ ID NO: 3 (corresponding to position 254 of SEQ ID NO: 1) was substituted with a lysine.
(b7) A serine at position 438 of SEQ ID NO: 3 (corresponding to position 438 of SEQ ID NO: 1) was substituted with a lysine.

The amino acid sequence set forth in SEQ ID NO: 12 is an amino acid sequence modified from the amino acid sequence set forth in SEQ ID NO: 4. The amino acid sequence set forth in SEQ ID NO: 4 and the amino acid sequence set forth in SEQ ID NO: 1 were aligned with each other, and amino acids were substituted as described below to give the amino acid sequence set forth in SEQ ID NO: 12.

(b1) A glutamic acid at position 105 of SEQ ID NO: 4 (corresponding to position 112 of SEQ ID NO: 1) was substituted with a lysine.
(b7) An arginine at position 433 of SEQ ID NO: 4 (corresponding to position 438 of SEQ ID NO: 1) was substituted with a lysine.

The amino acid sequence set forth in SEQ ID NO: 13 is an amino acid sequence modified from the amino acid sequence set forth in SEQ ID NO: 5. The amino acid sequence set forth in SEQ ID NO: 5 and the amino acid sequence set forth in SEQ ID NO: 1 were aligned with each other, and amino acids were substituted as described below to give the amino acid sequence set forth in SEQ ID NO: 13.

(b1) A glutamic acid at position 109 of SEQ ID NO: 5 (corresponding to position 112 of SEQ ID NO: 1) was substituted with a lysine.

(b3) A glycine at position 134 of SEQ ID NO: 5 (corresponding to position 137 of SEQ ID NO: 1) was substituted with a lysine.

(b4) A proline at position 244 of SEQ ID NO: 5 (corresponding to position 247 of SEQ ID NO: 1) was substituted with a lysine.

(b5) An arginine at position 251 of SEQ ID NO: 5 (corresponding to position 254 of SEQ ID NO: 1) was substituted with a lysine.

(b7) An asparagine at position 434 of SEQ ID NO: 5 (corresponding to position 438 of SEQ ID NO: 1) was substituted with a lysine.

The amino acid sequence set forth in SEQ ID NO: 14 is an amino acid sequence modified from the amino acid sequence set forth in SEQ ID NO: 2. The amino acid sequence set forth in SEQ ID NO: 2 and the amino acid sequence set forth in SEQ ID NO: 1 were aligned with each other, and amino acids were substituted as described below to give the amino acid sequence set forth in SEQ ID NO: 14.

(c1) An arginine at position 34 of SEQ ID NO: 2 (corresponding to position 34 of SEQ ID NO: 1) was substituted with a lysine.

(c2) A lysine at position 37 of SEQ ID NO: 2 (corresponding to position 37 of SEQ ID NO: 1) was substituted with a threonine.

(c3) A glutamine at position 177 of SEQ ID NO: 2 (corresponding to position 177 of SEQ ID NO: 1) was substituted with a lysine.

(c4) A lysine at position 198 of SEQ ID NO: 2 (corresponding to position 198 of SEQ ID NO: 1) was substituted with a glutamic acid.

(c5) An aspartic acid at position 297 of SEQ ID NO: 2 (corresponding to position 297 of SEQ ID NO: 1) was substituted with a lysine.

(c6) A lysine at position 302 of SEQ ID NO: 2 (corresponding to position 302 of SEQ ID NO: 1) was substituted with a valine.

(c8) An arginine at position 424 of SEQ ID NO: 2 (corresponding to position 424 of SEQ ID NO: 1) was substituted with a lysine.

(c9) An aspartic acid at position 430 of SEQ ID NO: 2 (corresponding to position 430 of SEQ ID NO: 1) was substituted with a lysine.

(c10) A lysine at position 432 of SEQ ID NO: 2 (corresponding to position 432 of SEQ ID NO: 1) was substituted with a glycine.

(c11) A methionine at position 443 of SEQ ID NO: 2 (corresponding to position 443 of SEQ ID NO: 1) was substituted with a lysine.

The amino acid sequence set forth in SEQ ID NO: 15 is an amino acid sequence modified from the amino acid sequence set forth in SEQ ID NO: 3. The amino acid sequence set forth in SEQ ID NO: 3 and the amino acid sequence set forth in SEQ ID NO: 1 were aligned with each other, and amino acids were substituted as described below to give the amino acid sequence set forth in SEQ ID NO: 15.

(c2) A lysine at position 37 of SEQ ID NO: 3 (corresponding to position 37 of SEQ ID NO: 1) was substituted with a threonine.

(c3) A glutamine at position 177 of SEQ ID NO: 3 (corresponding to position 177 of SEQ ID NO: 1) was substituted with a lysine.

(c4) A lysine at position 198 of SEQ ID NO: 3 (corresponding to position 198 of SEQ ID NO: 1) was substituted with a glutamic acid.

(c5) An aspartic acid at position 297 of SEQ ID NO: 3 (corresponding to position 297 of SEQ ID NO: 1) was substituted with a lysine.

(c6) A lysine at position 302 of SEQ ID NO: 3 (corresponding to position 302 of SEQ ID NO: 1) was substituted with a valine.

(c8) An arginine at position 424 of SEQ ID NO: 3 (corresponding to position 424 of SEQ ID NO: 1) was substituted with a lysine.

(c9) An aspartic acid at position 430 of SEQ ID NO: 3 (corresponding to position 430 of SEQ ID NO: 1) was substituted with a lysine.

(c10) A lysine at position 432 of SEQ ID NO: 3 (corresponding to position 432 of SEQ ID NO: 1) was substituted with a glycine.

(c11) A methionine at position 443 of SEQ ID NO: 3 (corresponding to position 443 of SEQ ID NO: 1) was substituted with a lysine.

The amino acid sequence set forth in SEQ ID NO: 16 is an amino acid sequence modified from the amino acid sequence set forth in SEQ ID NO: 4. The amino acid sequence set forth in SEQ ID NO: 4 and the amino acid sequence set forth in SEQ ID NO: 1 were aligned with each other, and amino acids were substituted as described below to give the amino acid sequence set forth in SEQ ID NO: 16.

(c5) An asparagine at position 292 of SEQ ID NO: 4 (corresponding to position 297 of SEQ ID NO: 1) was substituted with a lysine.

(c6) A lysine at position 297 of SEQ ID NO: 4 (corresponding to position 302 of SEQ ID NO: 1) was substituted with a valine.

(c9) A glutamic acid at position 425 of SEQ ID NO: 4 (corresponding to position 430 of SEQ ID NO: 1) was substituted with a lysine.

(c11) A serine at position 438 of SEQ ID NO: 4 (corresponding to position 443 of SEQ ID NO: 1) was substituted with a lysine.

The amino acid sequence set forth in SEQ ID NO: 17 is an amino acid sequence modified from the amino acid sequence set forth in SEQ ID NO: 5. The amino acid sequence set forth in SEQ ID NO: 5 and the amino acid sequence set forth in SEQ ID NO: 1 were aligned with each other, and amino acids were substituted as described below to give the amino acid sequence set forth in SEQ ID NO: 17.

(c5) A glycine at position 294 of SEQ ID NO: 5 (corresponding to position 297 of SEQ ID NO: 1) was substituted with a lysine.

(c8) A glutamic acid at position 426 of SEQ ID NO: 5 (corresponding to position 430 of SEQ ID NO: 1) was substituted with a lysine.

(c10) A proline at position 439 of SEQ ID NO: 5 (corresponding to position 443 of SEQ ID NO: 1) was substituted with a lysine.

The amino acid sequence set forth in SEQ ID NO: 18 is an amino acid sequence modified from the amino acid sequence set forth in SEQ ID NO: 2. The amino acid sequence set forth in SEQ ID NO: 2 and the amino acid sequence set forth in SEQ ID NO: 1 were aligned with each other, and amino acids were substituted as described below to give the amino acid sequence set forth in SEQ ID NO: 18.

(a1) A lysine at position 61 of SEQ ID NO: 2 (corresponding to position 61 of SEQ ID NO: 1) was substituted with an arginine.

(a2) A lysine at position 67 of SEQ ID NO: 2 (corresponding to position 67 of SEQ ID NO: 1) was substituted with a glutamic acid.

(a3) An alanine at position 128 of SEQ ID NO: 2 (corresponding to position 128 of SEQ ID NO: 1) was substituted with a lysine.

(a4) An arginine at position 157 of SEQ ID NO: 2 (corresponding to position 157 of SEQ ID NO: 1) was substituted with a lysine.

(a5) An arginine at position 166 of SEQ ID NO: 2 (corresponding to position 166 of SEQ ID NO: 1) was substituted with a lysine.

(a6) A lysine at position 391 of SEQ ID NO: 2 (corresponding to position 391 of SEQ ID NO: 1) was substituted with a histidine.

(b1) A glutamic acid at position 112 of SEQ ID NO: 2 (corresponding to position 112 of SEQ ID NO: 1) was substituted with a lysine.

(b2) A lysine at position 115 of SEQ ID NO: 2 (corresponding to position 115 of SEQ ID NO: 1) was substituted with a glutamic acid.

(b3) A glutamine at position 137 of SEQ ID NO: 2 (corresponding to position 137 of SEQ ID NO: 1) was substituted with a lysine.

(b4) An arginine at position 247 of SEQ ID NO: 2 (corresponding to position 247 of SEQ ID NO: 1) was substituted with a lysine.

(b5) An aspartic acid at position 254 of SEQ ID NO: 2 (corresponding to position 254 of SEQ ID NO: 1) was substituted with a lysine.

(b7) An arginine at position 438 of SEQ ID NO: 2 (corresponding to position 438 of SEQ ID NO: 1) was substituted with a lysine.

(c1) An arginine at position 34 of SEQ ID NO: 2 (corresponding to position 34 of SEQ ID NO: 1) was substituted with a lysine.

(c2) A lysine at position 37 of SEQ ID NO: 2 (corresponding to position 37 of SEQ ID NO: 1) was substituted with a threonine.

(c3) A glutamine at position 177 of SEQ ID NO: 2 (corresponding to position 177 of SEQ ID NO: 1) was substituted with a lysine.

(c4) A lysine at position 198 of SEQ ID NO: 2 (corresponding to position 198 of SEQ ID NO: 1) was substituted with a glutamic acid.

(c5) An aspartic acid at position 297 of SEQ ID NO: 2 (corresponding to position 297 of SEQ ID NO: 1) was substituted with a lysine.

(c6) A lysine at position 302 of SEQ ID NO: 2 (corresponding to position 302 of SEQ ID NO: 1) was substituted with a valine.

(c8) An arginine at position 424 of SEQ ID NO: 2 (corresponding to position 424 of SEQ ID NO: 1) was substituted with a lysine.

(c9) An aspartic acid at position 430 of SEQ ID NO: 2 (corresponding to position 430 of SEQ ID NO: 1) was substituted with a lysine.

(c10) A lysine at position 432 of SEQ ID NO: 2 (corresponding to position 432 of SEQ ID NO: 1) was substituted with a glycine.

(c11) A methionine at position 443 of SEQ ID NO: 2 (corresponding to position 443 of SEQ ID NO: 1) was substituted with a lysine.

The amino acid sequence set forth in SEQ ID NO: 19 is an amino acid sequence modified from the amino acid sequence set forth in SEQ ID NO: 3. The amino acid sequence set forth in SEQ ID NO: 3 and the amino acid sequence set forth in SEQ ID NO: 1 were aligned with each other, and amino acids were substituted as described below to give the amino acid sequence set forth in SEQ ID NO: 19.

(a1) A lysine at position 61 of SEQ ID NO: 3 (corresponding to position 61 of SEQ ID NO: 1) was substituted with an arginine.

(a2) A lysine at position 67 of SEQ ID NO: 3 (corresponding to position 67 of SEQ ID NO: 1) was substituted with a glutamic acid.

(a3) An alanine at position 128 of SEQ ID NO: 3 (corresponding to position 128 of SEQ ID NO: 1) was substituted with a lysine.

(a4) An arginine at position 157 of SEQ ID NO: 3 (corresponding to position 157 of SEQ ID NO: 1) was substituted with a lysine.

(a5) An arginine at position 166 of SEQ ID NO: 3 (corresponding to position 166 of SEQ ID NO: 1) was substituted with a lysine.

(a6) A lysine at position 391 of SEQ ID NO: 3 (corresponding to position 391 of SEQ ID NO: 1) was substituted with a histidine.

(b 1) A glutamic acid at position 112 of SEQ ID NO: 3 (corresponding to position 112 of SEQ ID NO: 1) was substituted with a lysine.

(b2) A lysine at position 115 of SEQ ID NO: 3 (corresponding to position 115 of SEQ ID NO: 1) was substituted with a glutamic acid.

(b3) A glutamine at position 137 of SEQ ID NO: 3 (corresponding to position 137 of SEQ ID NO: 1) was substituted with a lysine.

(b4) An arginine at position 247 of SEQ ID NO: 3 (corresponding to position 247 of SEQ ID NO: 1) was substituted with a lysine.

(b5) A glutamic acid at position 254 of SEQ ID NO: 3 (corresponding to position 254 of SEQ ID NO: 1) was substituted with a lysine.

(b7) A serine at position 438 of SEQ ID NO: 3 (corresponding to position 438 of SEQ ID NO: 1) was substituted with a lysine.

(c2) A lysine at position 37 of SEQ ID NO: 3 (corresponding to position 37 of SEQ ID NO: 1) was substituted with a threonine.

(c3) A glutamine at position 177 of SEQ ID NO: 3 (corresponding to position 177 of SEQ ID NO: 1) was substituted with a lysine.

(c4) A lysine at position 198 of SEQ ID NO: 3 (corresponding to position 198 of SEQ ID NO: 1) was substituted with a glutamic acid.

(c5) An aspartic acid at position 297 of SEQ ID NO: 3 (corresponding to position 297 of SEQ ID NO: 1) was substituted with a lysine.

(c6) A lysine at position 302 of SEQ ID NO: 3 (corresponding to position 302 of SEQ ID NO: 1) was substituted with a valine.

(c8) An arginine at position 424 of SEQ ID NO: 3 (corresponding to position 424 of SEQ ID NO: 1) was substituted with a lysine.

(c9) An aspartic acid at position 430 of SEQ ID NO: 3 (corresponding to position 430 of SEQ ID NO: 1) was substituted with a lysine.

(c10) A lysine at position 432 of SEQ ID NO: 3 (corresponding to position 432 of SEQ ID NO: 1) was substituted with a glycine.

(c11) A methionine at position 443 of SEQ ID NO: 3 (corresponding to position 443 of SEQ ID NO: 1) was substituted with a lysine.

The amino acid sequence set forth in SEQ ID NO: 20 is an amino acid sequence modified from the amino acid sequence set forth in SEQ ID NO: 4. The amino acid sequence set forth in SEQ ID NO: 4 and the amino acid sequence set forth in SEQ ID NO: 1 were aligned with each other, and amino acids were substituted as described below to give the amino acid sequence set forth in SEQ ID NO: 20.

(a1) A lysine at position 60 of SEQ ID NO: 4 (corresponding to position 61 of SEQ ID NO: 1) was substituted with an arginine.

(a5) An arginine at position 159 of SEQ ID NO: 4 (corresponding to position 166 of SEQ ID NO: 1) was substituted with a lysine.

(b1) A glutamic acid at position 105 of SEQ ID NO: 4 (corresponding to position 112 of SEQ ID NO: 1) was substituted with a lysine.

(b6) An arginine at position 433 of SEQ ID NO: 4 (corresponding to position 438 of SEQ ID NO: 1) was substituted with a lysine.

(c5) An asparagine at position 292 of SEQ ID NO: 4 (corresponding to position 297 of SEQ ID NO: 1) was substituted with a lysine.

(c6) A lysine at position 297 of SEQ ID NO: 4 (corresponding to position 302 of SEQ ID NO: 1) was substituted with a valine.

(c9) A glutamic acid at position 425 of SEQ ID NO: 4 (corresponding to position 430 of SEQ ID NO: 1) was substituted with a lysine.

(c11) A serine at position 438 of SEQ ID NO: 4 (corresponding to position 443 of SEQ ID NO: 1) was substituted with a lysine.

The amino acid sequence set forth in SEQ ID NO: 21 is an amino acid sequence modified from the amino acid sequence set forth in SEQ ID NO: 5. The amino acid sequence set forth in SEQ ID NO: 5 and the amino acid sequence set forth in SEQ ID NO: 1 were aligned with each other, and amino acids were substituted as described below to give the amino acid sequence set forth in SEQ ID NO: 21.

(a4) A proline at position 154 of SEQ ID NO: 5 (corresponding to position 157 of SEQ ID NO: 1) was substituted with a lysine.

(a5) An arginine at position 163 of SEQ ID NO: 5 (corresponding to position 166 of SEQ ID NO: 1) was substituted with a lysine.

(a6) A lysine at position 387 of SEQ ID NO: 5 (corresponding to position 391 of SEQ ID NO: 1) was substituted with a histidine.

(b1) A glutamic acid at position 109 of SEQ ID NO: 5 (corresponding to position 112 of SEQ ID NO: 1) was substituted with a lysine.

(b3) A glycine at position 134 of SEQ ID NO: 5 (corresponding to position 137 of SEQ ID NO: 1) was substituted with a lysine.

(b4) A proline at position 244 of SEQ ID NO: 5 (corresponding to position 247 of SEQ ID NO: 1) was substituted with a lysine.

(b5) An arginine at position 251 of SEQ ID NO: 5 (corresponding to position 254 of SEQ ID NO: 1) was substituted with a lysine.

(b7) An asparagine at position 434 of SEQ ID NO: 5 (corresponding to position 438 of SEQ ID NO: 1) was substituted with a lysine.

(c5) A glycine at position 294 of SEQ ID NO: 5 (corresponding to position 297 of SEQ ID NO: 1) was substituted with a lysine.

(c9) A glutamic acid at position 426 of SEQ ID NO: 5 (corresponding to position 430 of SEQ ID NO: 1) was substituted with a lysine.

(c11) A proline at position 439 of SEQ ID NO: 5 (corresponding to position 443 of SEQ ID NO: 1) was substituted with a lysine.

Tables 3 to 6 show the SEQ ID NOs of the amino acid sequences of the pre-modification fructosyl amino acid oxidases and the post-modification fructosyl amino acid oxidases, substituted amino acids in the post-modification amino acid sequences, and their positions. For example, an arginine (R) at position 34 substituted with a lysine (K) is denoted as "R34K".

[Table 3]

| SEQ ID NO: | Amino Acid Substitutions |
|---|---|
| 2 | None |
| 6 | K61R K67E A128K R157K R166K K391H |
| 10 | E112K K115E Q137K R247K D254K R438K |
| 14 | R34K K37T Q177K K198E D297K K302V R424K D430K K432G M443K |
| 18 | R34K K37T K61R K67E E112K K115E A128K Q137K R157K R166K Q177K K198E R247K D254K D297K K302V K391H R424K D430K K432G R438K M443K |

[Table 4]

| SEQ ID NO: | Amino Acid Substitutions |
|---|---|
| 3 | None |
| 7 | K61R K67E A128K R157K R166K K391H |
| 11 | EL112K K115E Q137K R247K E254K S438K |
| 15 | K37T Q177K K198E D297K K302V R424K D430K K432G M443K |
| 19 | K37T K61R K67E E112K K115E A128K Q137K R157K R166K Q177K K198E R247K E254K D297K K302V K391H R424K D430K K432G S438K M443K |

[Table 5]

| SEQ ID NO: | Amino Acid Substitutions |
|---|---|
| 4 | None |
| 8 | K60R R159K |
| 12 | E105K R433K |
| 16 | N292K K297V E425K S438K |
| 20 | K60R E105K R159K N292K K297V E425K R433K S438K |

[Table 6]

| SEQ ID NO: | Amino Acid Substitutions |
|---|---|
| 5 | None |
| 9 | P154K R163K K387H |
| 13 | E109K G134K P244K R251K N434K |
| 17 | G294K E426K P439K |

(continued)

| SEQ ID NO: | Amino Acid Substitutions |
|---|---|
| 21 | E109K G134K P154K R163K P244K R251K G294K K387H E426K N434K P439K |

The amino acid sequence set forth in SEQ ID NO: 22 is an amino acid sequence modified from the amino acid sequence (SEQ ID NO: 24) of a known FAOD (Accession Number BAD54824) derived from Aspergillus oryzae. The amino acid sequence set forth in SEQ ID NO: 24 and the amino acid sequence set forth in SEQ ID NO: 1 were aligned with each other, and amino acids were substituted as described below to give the amino acid sequence set forth in SEQ ID NO: 22.

(b4) An arginine at position 247 of SEQ ID NO: 24 (corresponding to position 247 of SEQ ID NO: 1) was substituted with a lysine (R247K).
(b6) An asparagine at position 277 of SEQ ID NO: 24 (corresponding to position 277 of SEQ ID NO: 1) was substituted with a lysine (N277K).

The amino acid sequence set forth in SEQ ID NO: 23 is an amino acid sequence modified from the amino acid sequence (SEQ ID NO: 25) of a known FAOD (Accession Number AAB88209) derived from Aspergillus fumigatus. The amino acid sequence set forth in SEQ ID NO: 25 and the amino acid sequence set forth in SEQ ID NO: 1 were aligned with each other, and amino acids were substituted as described below to give the amino acid sequence set forth in SEQ ID NO: 23.

(b4) A glutamine at position 247 of SEQ ID NO: 25 (corresponding to position 247 of SEQ ID NO: 1) was substituted with a lysine (Q247K).
(c7) An arginine at position 413 of SEQ ID NO: 25 (corresponding to position 413 of SEQ ID NO: 1) was substituted with a lysine (R413K).
(c8) An arginine at position 424 of SEQ ID NO: 25 (corresponding to position 424 of SEQ ID NO: 1) was substituted with a lysine (R424K).
(c9) A glutamine at position 430 of SEQ ID NO: 25 (corresponding to position 430 of SEQ ID NO: 1) was substituted with a lysine (Q430K).
(c11) An arginine at position 443 of SEQ ID NO: 25 (corresponding to position 443 of SEQ ID NO: 1) was substituted with a lysine (R443K).

The fructosyl amino acid oxidases having amino acids modified as described above are those in which a thermal stability exhibited while they are immobilized on a support is improved compared to that of the pre-modification fructosyl amino acid oxidases.
The present invention can use a recombinant protein of a fructosyl amino acid oxidase consisting of any of these amino acid sequences. Specifically, the fructosyl amino acid oxidase that can be used in the present invention can be obtained as follows. A host cell transfected with an expression vector including a nucleotide sequence that encodes these amino acid sequences is provided. Examples of the host cell include Escherichia coli bacteria, yeasts, such as Saccharomyces cerevisiae and Pichia pastoris, mammalian cells, and insect cells. The host cell is cultured, and then, treatments, such as removal of bacteria or cells and purification and concentration of the protein are performed.
Another embodiment of the present invention is a fructosyl amino acid oxidase comprising an amino acid sequence that is identical to the amino acid sequence set forth in any of SEQ ID NOs: 1 to 23 except that one or more amino acids are modified or mutated, or deleted, substituted, added, and/or inserted, the fructosyl amino acid oxidase having physicochemical properties such as a high thermal stability exhibited while the fructosyl amino acid oxidase is immobilized on a support and a high substrate specificity for a particular glycated amino acid or glycated peptide. The one or more amino acids are 1 to 15, 1 to 10, 1 to 5, 1 to 4, 1 to 3, or 1 or 2 amino acids.
Furthermore, the present invention provides a glycated protein sensor. The glycated protein sensor of the present invention includes at least a support and an enzyme immobilized on the support. The enzyme includes at least a fructosyl amino acid oxidase. The glycated protein sensor of the present invention may be a glycated protein sensor that calculates an amount of a glycated protein present in a test sample, that is, a concentration of a glycated protein in a test sample.
A preferred embodiment of the glycated protein sensor of the present invention includes a support, a fructosyl amino acid oxidase immobilized on the support, and a detector. The detector detects hydrogen peroxide produced from a glycated amino acid and/or a glycated peptide by using the fructosyl amino acid oxidase. The glycated protein sensor can calculate the amount (concentration) of the glycated protein from the results of the detection performed by the detector.
In one embodiment of the glycated protein sensor of the present invention, the detector is a hydrogen peroxide detector that detects hydrogen peroxide, and specifically, the detector may be a hydrogen peroxide electrode. During the decomposition of the hydrogen peroxide into oxygen, electrons are emitted, and the hydrogen peroxide electrode detects the electrons as a current and, accordingly, can calculate the amount (concentration) of the hydrogen peroxide from the detected current value.

In another embodiment, the detector is an optical detector and detects hydrogen peroxide by measuring an absorbance or an amount of light. For example, the amount of hydrogen peroxide can be determined by causing a color reaction in the presence of a peroxidase and an oxidative coloring agent or detecting a luminol emission intensity.

In another embodiment, the detector is an electrochemiluminescence detector in which a gold electrode, a platinum electrode, or a transparent electrode made of indium tin oxide (ITO electrode) is used. Hydrogen peroxide is detected by measuring the emission from a luminescent reagent, such as luminol. The detector may be a hydrogen peroxide detector that uses a different method.

The test sample for the glycated protein sensor of the present invention may be a solution. The solution may be a body fluid, a solution that is body-fluid-derived, or a diluted solution of a body fluid. The solution may be a solution that is not a body fluid (not body-fluid-derived) or may be a liquid mixture of a body fluid or a solution that is body-fluid-derived and a solution that is not body-fluid-derived. The solution may be a solution for use in a measurement of samples or a solution for use in a measurement for calibration. For example, the solution may be a standard solution or a calibration solution. The solution may include a buffer solution.

The body fluid may be blood, a serum, a plasma, or a lymph fluid, may be a tissue fluid, such as an intercellular fluid, a transcellular fluid, or an interstitial fluid, or may be a body cavity fluid, a serous cavity fluid, a pleural fluid, an ascites fluid, a pericardial fluid, a cerebrospinal fluid (spinal fluid), a joint fluid (synovial fluid), or an eye's aqueous humor (aqueous humor). The body fluid may be a digestive fluid, such as saliva, gastric juice, bile, pancreatic juice, or an intestinal fluid, or may be sweat, tears, nasal mucus, urine, semen, a vaginal fluid, an amniotic fluid, or milk. The body fluid may be an animal body fluid or a human body fluid. The body fluid may be a liquid present in food containing an animal-derived protein (e.g., milk or a dairy product). The body fluid may be a plant body fluid, a plant biological fluid, or a plant-derived liquid. For example, the body fluid may be a plant juice, a nectar, or a sap.

The solution may contain a measurement target substance. For example, the solution may be tears, and the measurement target substance may be albumin or glycoalbumin present in the tears. The measurement target substance may be albumin, glycoalbumin, hemoglobin, or glycohemoglobin present in blood, a serum, or a plasma, may be albumin or glycoalbumin present in an interstitial fluid, may be albumin or glycoalbumin present in urine, or may be albumin or glycoalbumin present in saliva.

A measurement target of the glycated protein sensor of the present invention is a glycated protein. The glycated protein may be a fructosamine. The "fructosamine" is a general term for glycated proteins present in the blood. Specific examples thereof include glycated albumin and glycated hemoglobin present in blood.

One embodiment of the present invention may further include a protease immobilized on a support, in addition to the fructosyl amino acid oxidase. The "protease" is a general term for peptide bond hydrolases that hydrolyze and catabolize a protein or a polypeptide. The protease may be an enzyme that degrades a protein into peptide fragments. In instances where a protein contains a glycated amino acid residue, the protease acts to produce one or more selected from the group consisting of a glycated amino acid, a glycated-amino-acid-containing peptide fragment, a non-glycated amino acid, and a peptide fragment with no glycated amino acid. Among these, a glycated amino acid or a glycated-amino-acid-containing peptide fragment reacts with the fructosyl amino acid oxidase, and, consequently, hydrogen peroxide can be produced.

In one embodiment of the present invention, a support on which the enzyme is immobilized forms a support layer, which is, for example, a thin film layer, and the support layer is layered and positioned on a detection surface of the detector. The support is positioned on or near the detection surface of the detector by using a coupling agent, such as a silane coupling agent. The formation of a coupling layer between the layered support and detector couples the support to the detection surface.

The coupling agent may be any of a variety of coupling agents as long as they do not substantially impair the measurement principle of the present disclosure. The coupling agent may contain, for example, a material that couples an inorganic material to an organic material. The coupling agent may be, for example, a silane coupling agent. Examples of the silane coupling agent include the following silane coupling agents:

vinyl-based: vinyltrimethoxysilane, vinyltriethoxysilane, 7-octenyltrimethoxysilane, vinyldimethylethoxysilane, vinylmethyldimethoxysilane, vinylmethyldiethoxysilane, vinyltris(2-methoxyethoxy)silane, vinyltris(trimethylsiloxy)silane, 4-vinylphenyltrimethoxysilane, allyltrimethoxysilane, allyltriethoxysilane, and 5-(triethoxysilyl)-2-norbornene;

styryl-based: p-styryltrimethoxysilane;

methacrylic-based: 3-methacryloxypropyl trimethoxysilane, 3-methacryloxypropylmethyl dimethoxysilane, 3-methacryloxypropyl triethoxysilane, 3-methacryloxypropylmethyl diethoxysilane, 3-methacryloxypropyl tris(trimethylsiloxy) silane, 3-methacryloxypropyl triallylsilane, and 8-methacryloxyoctyl trimethoxysilane;

acrylic-based: 3-acryloxypropyl trimethoxysilane, 3-acryloxypropyl dimethylmethoxysilane, and 3-acryloxypropyl triallylsilane;

epoxy-based: 3-glycidoxypropyltrimethoxysilane, 3-glycidoxypropylmethyl dimethoxysilane, 3-glycidoxypropylmethyl diethoxysilane, 8-glycidoxyoctyl trimethoxysilane, and 2-(3,4-epoxycyclohexyl)ethyltrimethoxysilane;

amino-based: N-2-(aminoethyl)-3-aminopropyltrimethoxysilane, N-2-(aminoethyl)-3-aminopropylmethyldimethoxysilane, N-2-(aminoethyl)-3-aminopropyltriethoxysilane, N-2-(aminoethyl)-8-aminooctyltrimethoxysilane, N-6-(aminohexyl)-3-aminopropyltrimethoxysilane, 3-aminopropyltrimethoxysilane, 3-aminopropylmethyldimethoxysilane, 3-aminopropyltriethoxysilane (APTES), 3-aminopropylmethyldiethoxysilane, 3-triethoxysilyl-N-(1,3-dimethyl-butylidene)propylamine, N-phenyl-3-aminopropyltrimethoxysilane, N-methyl-3-aminopropyltrimethoxysilane, N,N-dimethyl-3-aminopropyltrimethoxysilane, bis[3-(trimethoxysilyl)-propyl]amine, and N-(vinylbenzyl)-2-aminoethyl-3-aminopropyltrimethoxysilane hydrochloride;

ureide-based: 3-ureidepropyl trimethoxysilane and 3-ureidepropyl triethoxysilane; azide-based: 11-azideundecil trimethoxysilane;

isocyanate-based: 3-isocyanatepropyltrimethoxysilane and 3-isocyanatepropyltriethoxysilane; isocyanurate-based: tris-(trimethoxysilylpropyl) isocyanurate;

mercapto-based: 3-mercaptopropyltrimethoxysilane, 3-mercaptopropylmethyldimethoxysilane, and 3-mercaptopropyltriethoxysilane; and acid anhydride: 3-trimethoxysilylpropyl succinic anhydride.

Furthermore, the present invention provides a method for measuring a glycated protein. The method of the present invention for measuring a glycated protein detects a glycated protein by using a reaction of a fructosyl amino acid oxidase immobilized on a support.

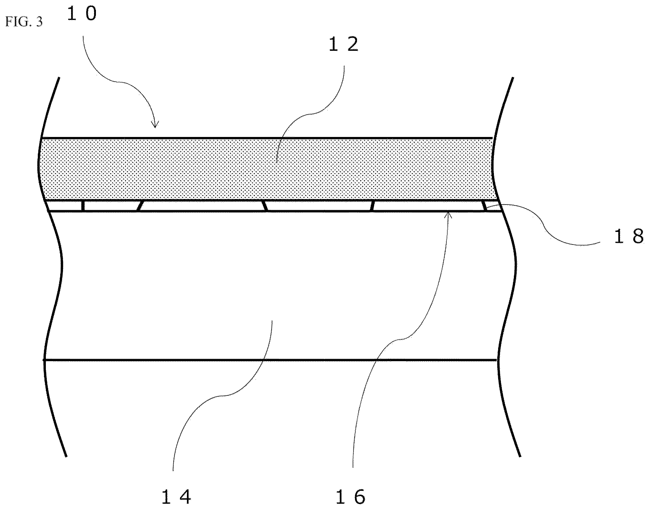

Fig. 3 illustrates a configuration of a glycated protein sensor 10, which is an embodiment of the present invention. The sensor 10, illustrated in Fig. 3, includes an enzyme layer 12 and a hydrogen peroxide detector 14. The enzyme layer 12 includes a fructosyl amino acid oxidase and a protease. The fructosyl amino acid oxidase and the protease have been crosslinked on bovine serum albumins, which are supports, by means of glutaraldehyde, which is a crosslinking agent. The bovine serum albumins have also been crosslinked with each other by means of glutaraldehyde. These form the enzyme layer 12. The enzyme layer 12 is layered on a detection surface 16-side of the hydrogen peroxide detector 14 and is coupled to the detection surface 16 of the hydrogen peroxide detector 14 by a silane coupling agent 18.

In another embodiment, a glycated protein sensor includes not only the enzyme layer and the hydrogen peroxide detector but also an additional layer. The additional layer is, for example, a permeation-limiting layer, and the permeation-limiting layer may contain a polycarbonate or an ion exchange resin. The permeation-limiting layer is provided, for example, outside the enzyme layer or between the enzyme layer and the hydrogen peroxide detector. The permeation-limiting layer may be coupled via hydrogen bonding, ionic bonding, or the like.

The ion exchange resin may be a cation exchange resin and/or an anion exchange resin. For example, using a cation exchange resin, such as a Nafion (registered trademark), can inhibit or prevent anions present in an analyte from reaching the detector. For example, using an anion exchange resin, such as a polypyrrole, can inhibit or prevent cations present in an analyte from reaching the detector. The permeation-limiting layer may contain one or more or at least one type of ion exchange resin and may be formed of one or more or at least one type of layer.

When a glycated protein that is a measurement target is introduced, the protease immobilized on the support degrades the glycated protein into glycated peptide fragments and non-glycated peptide fragments. These peptide fragments are believed to diffuse within the enzyme layer 12, and when glycated peptide fragments react with the immobilized fructosyl amino acid oxidase, hydrogen peroxide is produced.

The hydrogen peroxide detector 14 detects the hydrogen peroxide and outputs a signal associated with a concentration thereof. When the hydrogen peroxide detector 14 is a hydrogen peroxide electrode, the hydrogen peroxide is decomposed at the hydrogen peroxide electrode, and the emitted electrons are detected as a current.

The glycated protein sensor 10 includes a controller. The controller calculates the concentration of the glycated protein from the current detected by the hydrogen peroxide electrode. The controller can determine the concentration of the glycated protein present in the analyte solution based on a predetermined relationship between the concentrations of glycated proteins and the values of currents generated at the hydrogen peroxide electrode.

The fructosyl amino acid oxidase has a high thermal stability while being immobilized on a bovine serum albumin that is a support, and/or has a specific reactivity with fructosyl lysine. Accordingly, the glycated protein sensor 10 can accurately calculate the concentration of a particular glycated protein.

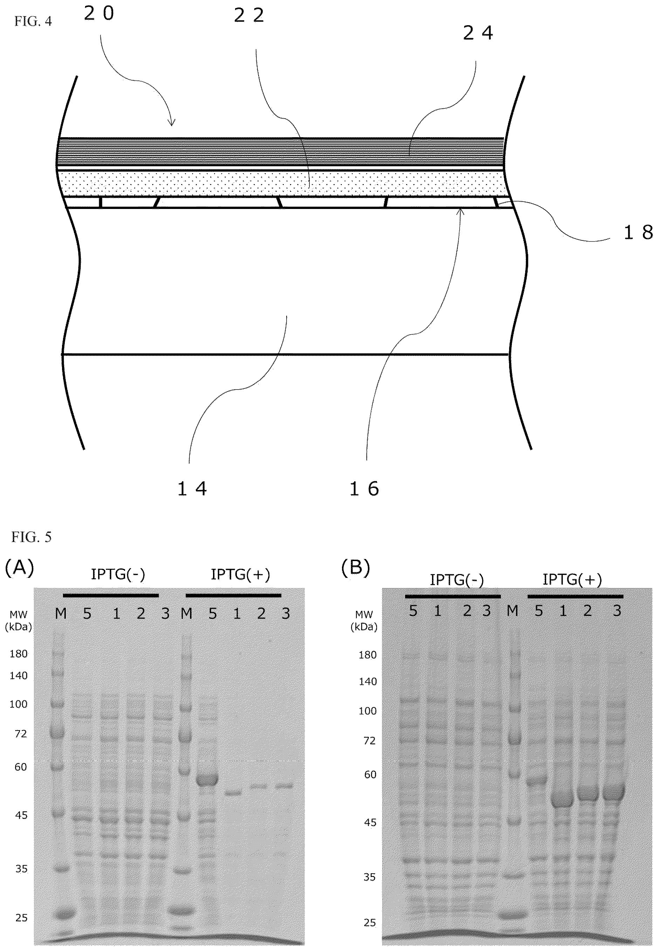

Fig. 4 illustrates another embodiment of the present invention. In a glycated protein sensor 20, a layer including a support on which a fructosyl amino acid oxidase is immobilized (fructosyl amino acid oxidase layer 22) is formed on the hydrogen peroxide detector 14. A layer including a support on which a protease is immobilized (protease layer 24) is formed on the fructosyl amino acid oxidase layer 22, that is, on a surface opposite to the hydrogen peroxide detector 14. That is, the fructosyl amino acid oxidase layer 22 and the protease layer 24 are layered in this order on the hydrogen peroxide detector 14.

In another embodiment, a glycated protein sensor includes not only the enzyme layer and the hydrogen peroxide detector but also an additional layer. The additional layer is, for example, a permeation-limiting layer as described above, which may be provided outside the fructosyl amino acid oxidase layer 22, between the fructosyl amino acid oxidase layer 22 and the protease layer 24, or between the fructosyl amino acid oxidase layer 22 and the hydrogen peroxide detector 14. The permeation-limiting layer may be coupled via hydrogen bonding, ionic bonding, or the like.

When a glycated protein that is a measurement target is introduced, the protease in the protease layer 24 degrades the glycated protein into glycated peptide fragments and non-glycated peptide fragments. These peptide fragments are believed to infiltrate into the fructosyl amino acid oxidase layer 22, and when glycated peptide fragments react with the immobilized fructosyl amino acid oxidase, hydrogen peroxide is produced, and the hydrogen peroxide detector 14 detects the produced hydrogen peroxide.

In another embodiment of the present invention, a glycated protein sensor includes the fructosyl amino acid oxidase layer but includes no protease immobilized on a support. The glycated protein sensor may be one in which a solution that has been protease-treated is introduced as the measurement target into the glycated protein sensor and in which the hydrogen peroxide detector detects hydrogen peroxide produced in the fructosyl amino acid oxidase layer.

EXAMPLES

The present invention will be described in more detail with reference to Examples. The present invention is not limited to the Examples described below.

The method used to produce fructosyl amino acid oxidases for use in the Examples will be described in detail below. First, genes designed to include respective nucleotide sequences for encoding the amino acid sequences set forth in SEQ ID NOs: 1 to 5 and to add a histidine tag to the N-terminus of each of the inserted proteins were introduced into respective expression vectors including a lactose operon. The expression vectors were used to transform an Escherichia coli bacteria BL21 (DE3) strain (Novagen, manufactured by Merck). Fructosyl amino acid oxidases produced from the respective Escherichia coli bacteria and consisting of the respective amino acid sequences set forth in SEQ ID NOs: 1 to 5 are hereinafter respectively referred to as Examples 1 to 5.

First, it was confirmed by SDS-PAGE that the target proteins could be produced from the Escherichia coli bacteria transfected with respective nucleotide sequences for encoding fructosyl amino acid oxidases consisting of the respective amino acid sequences set forth in SEQ ID NOs: 1 to 3 and 5.

Each of the Escherichia coli bacteria was cultured with shaking for 2.5 hours in an LB liquid medium containing 50 mg/mL of Kanamycin (manufactured by Nacalai Tesque, Inc.). In addition, each of the Escherichia coli bacteria was cultured overnight with shaking in an LB liquid medium, which was supplemented with isopropyl β-D-thiogalactopyranoside (hereinafter referred to as "IPTG") to a final concentration of 0.5 mM, and in an LB liquid medium not supplemented with IPTG. After the culturing, the OD600 was measured to confirm the growth of the Escherichia coli bacteria.

A cell lysis buffer (which contained a HEPES buffer solution at 98 vol.%, 100 mg/mL of a lysozyme solution at 1 vol.%, and a 10% Triton (registered trademark) X-100 solution at 1 vol.% and to which 0.5 M tris(2-carboxyethyl)phosphine at 1 vol.% and 10 U/μL DpnI at 0.2 vol.% were added) was added to a bacterial pellet obtained by centrifuging the culture medium, such that a uniform bacterial density could be achieved. Then, the resultant was incubated on ice for 1 hour. The resultant was centrifuged, and the supernatant was collected. The sediment was mixed with a HEPES buffer solution added thereto.

8 μL of a sample buffer solution (2ME+)(×2) (manufactured by Fujifilm Wako Pure Chemical Corporation) was added to and mixed with 8 μL of the supernatant and also added to and mixed with 8 μL of the sediment. The mixtures were heat-treated at 95°C for 10 minutes. The mixtures were applied to an SDS-PAGE gel (Perfect NT Gel M, manufactured by DRC Co., Ltd.) to be subjected to electrophoresis, which was performed at a constant voltage of 180 V. Staining was performed with Quick-CBB PLUS (manufactured by Fujifilm Wako Pure Chemical Corporation).

The results are shown in Fig. 5. Fig. 5 (A) shows the result of the electrophoresis of the supernatant, and Fig. 5 (B) shows that of the sediment. In the figures, "M" denotes a molecular weight marker, the number of each of the lanes denotes the SEQ ID NO of the protein introduced into the Escherichia coli bacteria, "IPTG (-)" denotes Escherichia coli bacteria cultured without supplementation with IPTG, and "IPTG (+)" denotes Escherichia coli bacteria cultured with supplementation with IPTG. Approximately 48 to 53 kDa of the protein was present in the supernatant and the sediment of the bacteria treated in the cell lysis solution supplemented with IPTG, and, accordingly, it was confirmed that recombinant fructosyl amino acid oxidases of Examples 1 to 3 and 5 were produced.

Furthermore, the introduced protein was purified from the transformed Escherichia coli bacteria. Each of the Escherichia coli bacteria was cultured in the above-described LB medium, and after the expression was induced with IPTG, the bacteria was cultured at 18°C for 20 hours. Then, the collected Escherichia coli bacteria were suspended in a buffer A (20 mM Tris-HCL (pH 7.5) containing 0.25 M sodium chloride and 20 mM imidazole), and the bacteria were then sonicated in a sonicator and centrifuged to give a homogenate supernatant. The homogenate supernatant was treated with a filter having a pore size of 0.45 μm and subsequently was applied to a HisTrap HP (GE Healthcare) and washed with the buffer A in an amount six times the volume of the column. Furthermore, elution was performed in an elution buffer (20

**EP 4 471 135 A1**

mM Tris-HCL (pH 7.5) containing 0.25 M sodium chloride and 0.5 M imidazole).

The resulting eluate was dialyzed against a 20 mM potassium phosphate buffer (pH 7.5) and sterilized with a filter having a pore size of 0.22 μm, to give a sample. The protein concentration of the sample was measured with a Protein Assay Kit I (manufactured by Bio-Rad Laboratories, Inc.). Table 7 shows the volume of the samples containing Examples 1 to 5 and the protein concentration of the samples.

[Table 7]

| Example | SEQ ID NO: | Volume (mL) | concentration (mg/mL) |
|---|---|---|---|
| 1 | 1 | 9 | 10.9 |
| 2 | 2 | 9 | 6.14 |
| 3 | 3 | 10 | 8.98 |
| 4 | 4 | 9 | 0.84 |
| 5 | 5 | 10 | 7.65 |

The activity of the fructosyl amino acid oxidases of Examples 1 to 5 was examined. Comparative Examples that were used were a fructosyl amino acid oxidase FAOD-E (manufactured by Kikkoman Corporation, Comparative Example 1), a Lucica (registered trademark) GA-L (manufactured by Asahi Kasei Pharma Corporation, Comparative Example 2), and a fructosyl amino acid oxidase consisting of the amino acid sequence set forth in SEQ ID NO: 26 (Comparative Example 3). SEQ ID NO: 26 is known as an FAD-dependent oxidoreductase (Accession Number KZZ92706) derived from Ascosphaera apis and has a homology of 55 to 63% to the amino acid sequence of a known fructosyl amino acid oxidase. The fructosyl amino acid oxidase of Comparative Example 1 was purified in a manner similar to that of Examples 1 to 5. The obtained protein solution was in an amount of 10 mL and had a concentration of 15.8 mg/mL.

The substrates used were fructosyl lysine (F-Lys), fructosyl valyl histidine (F-Val-His), fructosyl valine (F-Val), and fructosyl glycine (F-Gly). In Examples 1 to 5 and Comparative Examples 1 and 3, 20 μL of a substrate solution containing a 1 mM substrate was added to each of the wells of a microplate at room temperature; thereafter, 10 μL of a 0.5 wt.% oxidative colorimetric reagent TOOS (manufactured by Dojindo Laboratories), 260 μL of a solution containing 4.76 μg/mL of peroxidase and 0.1 mg/mL of 4-aminoantipyrine (manufactured by Nacalai Tesque, Inc.), a HEPES buffer solution (pH 8.0) containing 0.15 M sodium chloride, and 5 μL of an appropriately diluted solution of the fructosyl amino acid oxidase were added and mixed together; and thereafter, changes in the absorbance at 555 nm were measured under a condition of 37°C. A negative control used was a 20 mM potassium phosphate buffer (pH 7.5).

In Comparative Example 2, 240 μL of a pretreatment solution (GAR-1) was added to the wells, and 6 μL of the substrate was added. A reaction was allowed to take place at 37°C for 5 minutes. Subsequently, 60 μL of an enzyme solution (GA R-2) was added, and further, a reaction was allowed to take place at 37°C for 5 minutes. Immediately before the addition of the enzyme solution and 5 minutes after the addition, the absorbance was measured at a dominant wavelength of 546 nm and a complementary wavelength of 700 nm, for which purified water was used as a control. Accordingly, a change in the absorbance over the time period was determined.

In the Examples and Comparative Examples, an activity of the fructosyl amino acid oxidases was observed in instances in which the F-Lys was used as the substrate, and thus, the activity for each of the F-Val-His, F-Val, and F-Gly was measured under the same conditions used for the F-Lys. Tables 8 and 9 show the activity for F-Lys and the specific activity for each of F-Val-His, F-Val, and F-Gly of the Examples and Comparative Examples. It was confirmed that Examples 1 to 5 had a high substrate specificity for F-Lys.

[Table 8]

| Example | SEQ ID NO: | Specific Activity | | | |
|---|---|---|---|---|---|
| | | F-Lys | F-Val-His | F-Val | F-Gly |
| 1 | 1 | 100 | 0.8 | 0.8 | 0.4 |
| 2 | 2 | 100 | 0.7 | 0.6 | 0.6 |
| 3 | 3 | 100 | 0.1 | 0.0 | 0.0 |
| 4 | 4 | 100 | 0.8 | 0.0 | 0.0 |
| 5 | 5 | 100 | 1.7 | 0.6 | 0.0 |

27

[Table 9]

| Comparative Example | SEQ ID NO: | Specific Activity | | | |
|---|---|---|---|---|---|
| | | F-Lys | F-Val-His | F-Val | F-Gly |
| 1 | - | 100 | 0.0 | 54.0 | 7.0 |
| 2 | - | 100 | 0.0 | 60.0 | 43.0 |
| 3 | 26 | 100 | 32.1 | 74.1 | 24.9 |

An examination was performed on a thermal stability in a liquid phase and the thermal stability in an immobilized state of the fructosyl amino acid oxidase (Example 1) consisting of the amino acids set forth in SEQ ID NO: 1, which was purified in the manner described above, and on those of a Comparative Example, which was an FAOD-E (manufactured by Kikkoman Corporation, Comparative Example 1).

First, a comparison was made between the thermal stability in a liquid phase of the enzyme of Example 1 and that of the enzyme of Comparative Example 1. HEPES buffer solutions (hereinafter referred to as "diluted enzyme solutions") containing 1 U/mL of the respective enzymes were each dispensed in an amount of 80 μL into tubes, and the tubes were held on heat blocks at temperatures of 30°C, 40°C, 50°C, and 55°C for 15 minutes. Next, at room temperature, 10 μL of a 0.5 w/w% TOOS and 270 μL of a POD/4-AA solution (solution diluted with a HEPES buffer solution (pH 8.0, 0.15 M NaCl) such that the peroxidase was present in an amount of 4.76 μg/mL, and the 4-aminoantipyrine was present in an amount of 0.1 mg/mL) were added to the wells of a microtiter plate containing 20 μL of 1 mM F-Lys, and 5 μL of the diluted enzyme solution, which had been heat-treated, was also added thereto. These were mixed by pipetting. The absorbance at 555 nm was measured with a microtiter plate reader at a setting of 37°C, every 15 seconds for 10 minutes after the addition, and a change in the absorbance per unit time was determined as an enzyme activity. Ratios of the activities under the treatment conditions of 40°C, 50°C, and 55°C to the enzyme activity at 30°C were calculated as residual activities, assuming that the enzyme activity under the condition of the treatment at 30°C was 100%.

Furthermore, to verify the thermal stability exhibited by the enzyme while being immobilized on a support, a glycated protein sensor (Example) was prepared. A silane coupling treatment was performed on a platinum electrode to incorporate an amino group into a surface of the electrode. In addition, bovine serum albumin (BSA) was added to a TES buffer solution, the enzyme of Example 1 was added, and the solution was stirred. Furthermore, a predetermined amount of a glutaraldehyde solution was added to the mixed solution containing the enzyme and the BSA, and the solution was stirred. 1 μL of the solution was added dropwise to the platinum electrode having the amino group incorporated therein and was thoroughly dried. In this manner, the sensor of the Example was prepared. In a Comparative Example, the enzyme of Comparative Example 1 (FAOD-E, manufactured by Kikkoman Corporation) was used instead of the enzyme of Example 1.

200 μL of a HEPES buffer solution containing 50 μM F-Lys was added dropwise to the sensors of the Example and Comparative Example, and electric current output values were obtained. Each of the sensors was immersed in HEPES buffer solutions heated to respective temperatures of 50°C, 65°C, and 80°C, and the sensors were allowed to stand at the temperatures for 20 minutes. Subsequently, each of the sensors was allowed to stand at 4°C for 30 minutes so that an influence of residual heat could be eliminated. Electric output values for F-Lys were obtained in a manner similar to that of the measurement performed before the thermal treatment. A ratio of the electric output value after the thermal treatment to the electric output value before the thermal treatment was calculated as a residual activity, assuming that the electric output value before the thermal treatment was 100%.

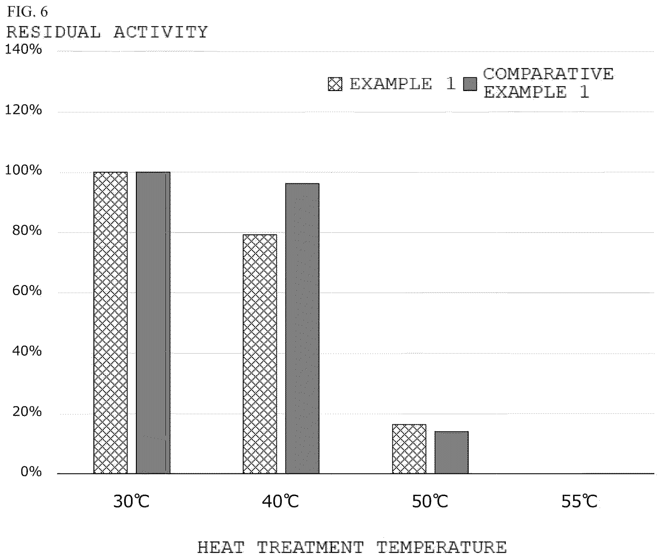

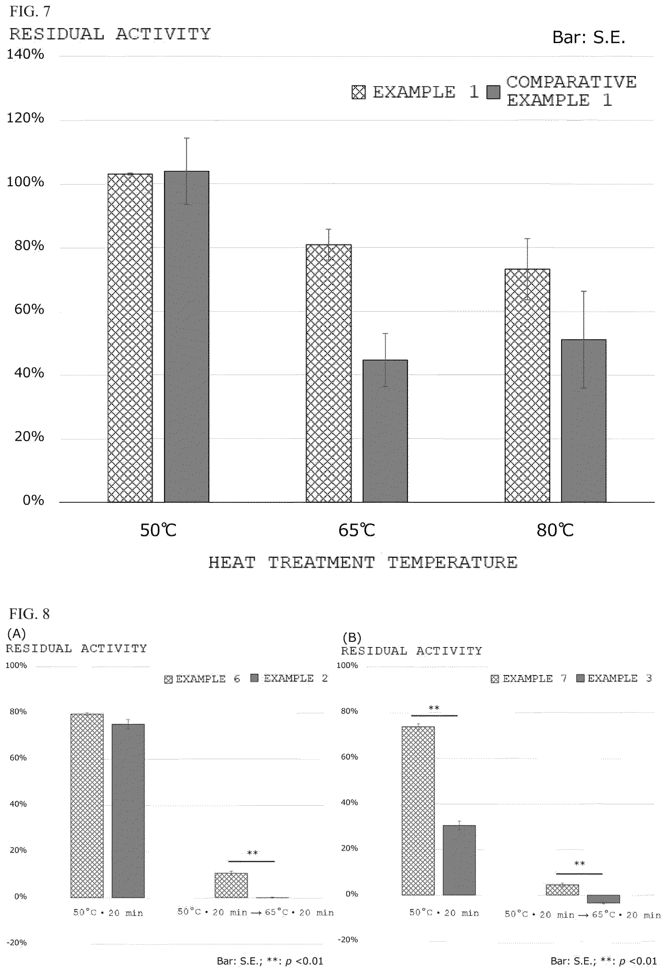

Fig. 6 shows the results of the measurements of thermal stabilities exhibited in the liquid phase, and Fig. 7 shows the results of the measurements of thermal stabilities exhibited in an immobilized state. In the liquid phase, the fructosyl amino acid oxidase of Example 1 exhibited a residual activity substantially comparable to that of the Comparative Example. On the other hand, in the state in which the enzyme was immobilized on a support, the enzyme exhibited a residual activity for F-Lys of 70% or greater even after the treatment at 65°C or 80°C for 20 minutes and, therefore, showed superiority over the Comparative Example.

Next, an examination was performed on the thermal stability of variants of fructosyl amino acid oxidases. Specifically, a fructosyl amino acid oxidase consisting of the amino acid sequence set forth in SEQ ID NO: 18 (hereinafter referred to as Example 6) and a fructosyl amino acid oxidase consisting of the amino acid sequence set forth in SEQ ID NO: 19 (hereinafter referred to as Example 7) were used. For a comparison of the thermal stability, the fructosyl amino acid oxidase consisting of the amino acid sequence set forth in SEQ ID NO: 2 (Example 2) and the fructosyl amino acid oxidase consisting of the amino acid sequence set forth in SEQ ID NO: 3 (Example 3) were used.

The method used to produce the fructosyl amino acid oxidases of the Examples will be described below. Expression vectors pET 28b (Novagen, from Merck) were transfected with respective nucleotide sequences for encoding the respective amino acid sequences and were transformed into an Escherichia coli bacteria BL21 (DE3) strain (Nippon Gene Co., Ltd.). The bacteria were inoculated in an LB medium supplemented with Kanamycin and cultured at 37°C overnight. An additional LB medium was added to perform pre-culture, and IPTG was added to a final concentration of 0.5 mM to induce expression overnight. The next day, pellets containing the respective enzymes were collected and frozen.

The frozen pellets were each suspended in an equilibration buffer (20 mM Tris-HCL (pH 8.4), 250 mM NaCl, and 20 mM imidazole), and thereafter, lysozyme and TCEP were added and further suspended. Triton X-100 was added and suspended, and subsequently, the suspension was sonicated. Thereafter, DNase I was added, and the resultant was shaken and centrifuged to collect the supernatant. The prepared sample was applied to a column and purified in an elution buffer (20 mM Tris-HCL (pH 8.4), 250 mM NaCl, and 20 mM imidazole). The resultant was dialyzed twice against a TES buffer (10 mM TES, 150 mM NaCl, pH 7.0). The confirmation that the target enzyme had been purified was made by using a micro-spectrophotometer.

To verify the thermal stability exhibited by the enzymes while being immobilized on a support, glycated protein sensors were prepared. A silane coupling treatment was performed on platinum electrodes to incorporate an amino group into a surface of the electrodes. In addition, bovine serum albumin (BSA) was added to TES buffer solutions, the respective enzymes were added, and the solutions were stirred. Furthermore, a predetermined amount of a glutaraldehyde solution was added to the mixed solutions containing the enzyme and the BSA, and the solutions were stirred. 1 μL of the respective solutions were added dropwise to the respective platinum electrodes having the amino group incorporated therein and were thoroughly dried. In this manner, the sensors were prepared.

200 μL of a HEPES buffer solution containing 50 μM F-Lys was added dropwise to each of the sensors, and current output values were obtained. Furthermore, electric output values for F-Lys were obtained under the following conditions: a condition of immersing each of the sensors for 20 minutes in a HEPES buffer solution heated at 50°C; and a condition of immersing each of the sensors for 20 minutes in the HEPES buffer solution heated at 65°C after the immersion for 20 minutes at 50°C. A ratio of the electric output value after the thermal treatment to the electric output value before the thermal treatment was calculated as a residual activity, assuming that the electric output value before the thermal treatment was 100%. A t-test was used for a statistical test.

Fig. 8(A) shows a comparison of the residual activity between Example 6 and Example 2, and Fig. 8(B) shows a comparison of the residual activity between Example 7 and Example 3. It is demonstrated that in Example 6, the residual activity exhibited by the enzyme after being heat-treated at a temperature of 65°C while being immobilized on a support was significantly higher than that of Example 2, which was an enzyme in which the amino acids had not yet been modified, and that in Example 7, the residual activity exhibited by the enzyme after being heat-treated at a temperature of 50°C to 65°C while being immobilized on a support was significantly higher than that of Example 3, which was an enzyme in which the amino acids had not yet been modified.

Examination (1) of Lysine Residues Relevant to Thermal Stability of FAOD of Example 1

The above-described results of the experiment on the variants confirmed that lysine residues of Example 1 impart thermal stability to FAODs. Accordingly, an experiment was performed to identify which lysine residue in Example 1 played an important role for the thermal stability of FAODs. Variants 1 to 3 were prepared in a manner similar to that described above. In variant 1 (SEQ ID NO: 27), lysines at positions 64, 84, 87, 128, 157, 166, 167, 394, and 397 of Example 1 were substituted with an arginine. In variant 2 (SEQ ID NO: 28), lysines at positions 112, 137, 152, 247, 254, 255, 267, 277, and 438 of Example 1 were substituted with an arginine. In variant 3 (SEQ ID NO: 29), lysines at positions 34, 177, 233, 234, 297, 309, 413, 424, 430, and 443 of Example 1 were substituted with an arginine. Furthermore, glycated protein sensors in which Example 1 and variants 1 to 3 were individually immobilized were prepared.

200 μL of a HEPES buffer solution containing 50 μM F-Lys was added dropwise to each of the sensors, and electric current output values 120 seconds after the addition of F-Lys were obtained. Furthermore, electric output values for F-Lys were obtained under the following conditions: a condition of immersing each of the sensors for 20 minutes in a HEPES buffer solution heated at 50°C; and a condition of immersing each of the sensors for 20 minutes in the HEPES buffer solution heated at 60°C after the immersion under the preceding condition. A ratio of the electric output value after the thermal treatment to the electric output value before the thermal treatment was calculated as a residual activity, assuming that the electric output value before the thermal treatment was 100%. One-way ANOVA and post hoc Bonferroni analysis was used for the statistical test.

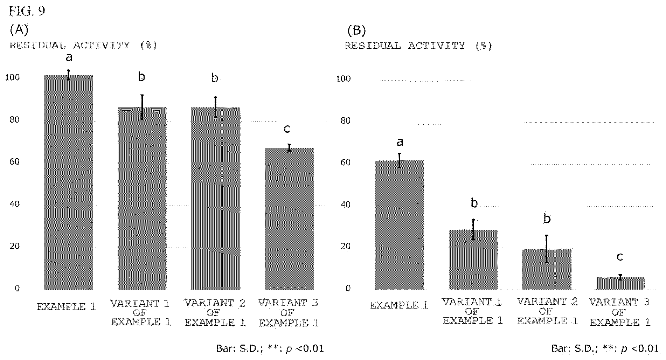

Fig. 9(A) shows the residual activity exhibited after the heating at 50°C, and Fig. 9(B) shows the residual activity exhibited after the heating at 60°C that followed the heating at 50°C. All of the variants exhibited a significantly lower residual activity than Example 1, which indicated that the lysines substituted by arginines in variants 1 to 3 played an important role for the thermal stability of Example 1. Furthermore, a comparison between the residual activities of the variants revealed that the residual activity of variant 3 was significantly lower than those of variants 1 and 2. This indicates that the lysines substituted by arginines in variant 3, in particular, were more important for the thermal stability of Example 1.

Examination of Method for Evaluating Thermal Stability of FAOD Being Immobilized on Support

To evaluate the thermal stability of FAODs being immobilized on a support, with an easier method, not by using an electrode, an examination was performed regarding changes in the thermal stability in a liquid phase exhibited by FAODs while being immobilized on a support and without being immobilized on a support. BSA was used as the support. The FAODs used were Examples 1 to 3 and an Example (hereinafter referred to as "Example 8") that was an FAOD consisting of the amino acid sequence set forth in SEQ ID NO: 20 and which was a variant of Example 3.

Furthermore, a variant of Example 1 (hereinafter referred to as "variant 4") was prepared. Variant 4 had an amino acid sequence identical to the amino acid sequence set forth in SEQ ID NO: 1, except that, (1) in an instance where the amino acid sequence set forth in SEQ ID NO: 2 and the amino acid sequence set forth in SEQ ID NO: 1 were aligned with each other, and an amino acid other than lysine was present at a position in the amino acid sequence set forth in SEQ ID NO: 1 while a lysine was present at a corresponding position in the amino acid sequence set forth in SEQ ID NO: 2, the amino acid other than lysine was substituted with a lysine; and (2) if a lysine was present at a position in the amino acid sequence set forth in SEQ ID NO: 1 while an amino acid other than lysine was present at a corresponding position in the amino acid sequence set forth in SEQ ID NO: 2, the lysine was substituted with an amino acid identical to the amino acid present at the corresponding position in the amino acid sequence set forth in SEQ ID NO: 2. The amino acid substitutions in variant 4 are as shown in Table 10, and the amino acid sequence is shown in SEQ ID NO: 30.

[Table 10]

| Variant 4: Amino Acid Substitutions in Amino Acid Sequence Set Forth in SEQ ID NO: 1 |
| --- |
| K34R T37K R61K E67K K112E E115K K128A K137Q K157R K166R K177Q E198K K247R K254D K297D V302K H391K K424R K430D G432K K438R K443M |

Hepes buffers (10 mM Hepes + 150 mM NaCl, pH 8.0) each containing 1 mg/mL of an FAOD (Example 1, Example 2, Example 8, and variant 3) and BSA were adjusted. In the case where the condition of immobilizing the FAOD onto the BSA was used, a predetermined amount of glutaraldehyde was further added. Incubation was performed at 25°C for 10 minutes, 90 μL of an ice-cold TAE buffer (40 mM Tris-acetate, 1 mM EDTA, pH 8.0 to 8.5) was added, and an excess amount of primary-amine-containing Tris was added to terminate the reaction. In this manner, an enzyme solution containing an FAOD that was immobilized onto BSA by the addition of glutaraldehyde was obtained, and an enzyme solution containing an FAOD that was not immobilized onto BSA as a result of not adding glutaraldehyde was obtained. The enzyme activity was measured after the enzyme solution was held on ice and after the enzyme solution was heated to 48°C on a heat block and held for 15 minutes. A ratio of the activity value obtained after the treatment at 48°C was calculated as the residual activity, assuming that the activity value of the enzyme solution held on ice was 100%.

To quantify an influence of the immobilization onto BSA on the residual activity of FAODs, based on the results of measurements of the residual activities exhibited without BSA immobilization and under BSA immobilization, a rate of change in the residual activity was calculated according to the following equation. The results are shown in Table 11.

[Math. 1]

$$\text{Rate of change in residual activity} = \frac{\text{Residual activity under BSA immobilization} - \text{Residual activity without BSA immobilization}}{\text{Residual activity under BSA immobilization}} \times 100$$

[Table 11]

| Mean Value ± Standard Deviation | Residual Activity Without BSA Immobilization (%) | Residual Activity Under BSA Immobilization (%) | Rate of Change in Residual Activity (%) |
| --- | --- | --- | --- |
| Example 1 | 24.3 ± 0.5 | 67.2 ± 6.5 | 176.7 |
| Example 2 | 21.0 ± 1.6 | 19.4 ± 1.5 | -7.8 |
| Example 3 | 38.9 ± 1.6 | 10.4 ± 0.2 | -73.2 |
| Example 8 | 14.6 ± 0.2 | 24.2 ± 0.2 | 65.9 |

(continued)

| Mean Value ± Standard Deviation | Residual Activity Without BSA Immobilization (%) | Residual Activity Under BSA Immobilization (%) | Rate of Change in Residual Activity (%) |
|---|---|---|---|
| Variant 4 | 48.7 ± 2.3 | 43.2 ± 0.1 | -11.4 |

As indicated, in the FAOD of Example 1, the residual activity exhibited under BSA immobilization was higher than those of the other FAODs, and the residual activity was significantly improved by the BSA immobilization compared to the residual activity exhibited without BSA immobilization. In contrast, the FAODs of Examples 2 and 3 had a reduced residual activity as a result of BSA immobilization. This tendency was the same as the results of the measurements performed by the glycated protein measurement sensors that used Example 2 or Example 3. Accordingly, this evaluation method can be used to estimate the thermal stability exhibited by an FAOD in the instance in which the FAOD is used in a glycated protein measurement sensor.

In variant 4, which is a variant of Example 1, the residual activity was reduced as a result of BSA immobilization, and in Example 8, which is a variant of Example 3, the residual activity was, on the contrary, improved as a result of BSA immobilization. Accordingly, it was indicated that lysine residues in Example 1 played an important role for the thermal stability of Example 1 that was exhibited under support immobilization. Furthermore, it was indicated that even if an FAOD different from that of Example 1 was used, the thermal stability that was exhibited under BSA immobilization could be improved by substituting the amino acid at a position corresponding to a lysine of Example 1 with a lysine.

Examination (2) of Lysine Residues Relevant to Thermal Stability of FAOD of Example 1

The results described above demonstrate that in the FAOD of Example 1, the lysines at positions 34, 177, 233, 234, 297, 309, 413, 424, 430, and 443 played a particularly important role for the thermal stability that was exhibited under BSA immobilization. Furthermore, these lysine residues were narrowed down to important lysine residues. Variant 5, variant 6, variant 7, and variant 8 were prepared in the same manner as that described above. Variant 5 (SEQ ID NO: 31) was identical to Example 1 except that the lysines at positions 233, 234, and 297 were substituted with an arginine. Variant 6 (SEQ ID NO: 32) was identical to Example 1 except that the lysines at positions 309 and 413 were substituted with an arginine. Variant 7 (SEQ ID NO: 33) was identical to Example 1 except that the lysines at positions 424, 430, and 443 were substituted with an arginine. Variant 8 (SEQ ID NO: 34) was identical to Example 1 except that the lysines at positions 34 and 177 were substituted with an arginine. Furthermore, the residual activities of each of the FAODs exhibited in a liquid phase under BSA immobilization and without BSA immobilization were measured in the same manner as that described above. In this experiment, the BSA concentration of the Hepes buffer was 2.64% (w/v).

Table 12 shows the residual activity exhibited without BSA immobilization, the residual activity exhibited under BSA immobilization, and the rate of change in the residual activity, of each of the FAODs. Variant 6 and variant 7 had a negative value of the rate of change in the residual activity, as opposed to Example 1 and other variants. The results indicate that the lysines substituted with an arginine in variant 6 and variant 7 played a particularly important role for the thermal stability of Example 1 exhibited under BSA immobilization. Accordingly, it is predicted that in the case of FAODs having a homology to Example 1, the thermal stability exhibited under BSA immobilization will be improved by substituting the amino acids corresponding to these positions of Example 1 with a lysine.

[Table 12]

| Mean Value ± Standard Deviation | Residual Activity Without BSA Immobilization (%) | Residual Activity Under BSA Immobilization (%) | Rate of Change in Residual Activity (%) |
|---|---|---|---|
| Example 1 | 50.8 | 62.4 | 22.8 |
| Variant 5 | 25.1 | 44.4 | 77.0 |
| Variant 6 | 59.4 | 49.2 | -17.1 |
| Variant 7 | 44.3 | 37.8 | -14.7 |
| Variant 8 | 37.3 | 46.4 | 24.2 |

Examination (4) of Lysine Residues Relevant to Thermal Stability of FAOD of Example 1

Comparative Example 4 (Accession Number BAD54824) is a known FAOD derived from Aspergillus oryzae. The amino acid sequence (SEQ ID NO: 24) of Comparative Example 4 has a high homology of 95.1% to the amino acid sequence of Example 1. In Comparative Example 4, the amino acid corresponding to the lysine at position 247 of Example 1 is an arginine, and the amino acid corresponding to the lysine at position 277 of Example 1 is an asparagine. Accordingly, analysis was performed on an influence of the lysines at positions 247 and/or 277 of Example 1 on the thermal stability of Example 1.

Variant 9 was prepared in the same manner as that described above, in addition to the FAODs of Example 1 and Comparative Example 4. Variant 9 (SEQ ID NO: 35) was identical to Example 1 except that the lysines at positions 247 and 277 were substituted with an arginine. The residual activity of each of the FAODs exhibited in a liquid phase under BSA immobilization was measured. One-way ANOVA and post hoc Bonferroni analysis was used for the statistical test. The results are shown in Table 13. The residual activity of Example 1 was significantly higher than those of Comparative Example 4 and variant 9, which indicated that the lysines at positions 247 and/or 277 of Example 1 played an important role for the thermal stability exhibited by FAODs while being immobilized.

[Table 13]

| Mean Value ± Standard Deviation | Residual Activity Under BSA Immobilization (%) |
|---|---|
| Example 1 | 62.4±1.6 [a] |
| Comparative Example 4 | 43.1±2.2 [b] |
| Variant 9 | 46.7±1.4 [b] |

The different alphabet indicates that the p-value was less than 0.001 in the statistical test.

Improvement in Thermal Stability of FAOD Based on Lysine Locations of Example 1

Comparative Example 5 (Accession Number AAB88209) is a known FAOD derived from Aspergillus fumigatus. The amino acid sequence (SEQ ID NO: 25) of Comparative Example 5 has a homology of 79.2% to the amino acid sequence of Example 1. An FAOD of Example 9 was prepared, which had an amino acid sequence identical to the amino acid sequence of Comparative Example 5 except that the following amino acid substitutions were included so that the thermal stability of the FAOD of Comparative Example 5 exhibited under support immobilization could be enhanced.

In Example 9, the amino acid sequence of Comparative Example 5 set forth in SEQ ID NO: 25 was identical to the amino acid sequence set forth in SEQ ID NO: 1, except that, (1) in an instance where the amino acid sequence set forth in SEQ ID NO: 25 and the amino acid sequence set forth in SEQ ID NO: 1 were aligned with each other, and an amino acid other than lysine was present at a position in the amino acid sequence set forth in SEQ ID NO: 25 while a lysine was present at a corresponding position in the amino acid sequence set forth in SEQ ID NO: 1, the amino acid other than lysine was substituted with a lysine; and (2) if a lysine was present at a position in the amino acid sequence set forth in SEQ ID NO: 1 while an amino acid other than lysine was present at a corresponding position in the amino acid sequence set forth in SEQ ID NO: 25, the lysine was substituted with an amino acid identical to the amino acid present at the corresponding position in the amino acid sequence set forth in SEQ ID NO: 25. The amino acid substitutions in the FAOD of Example 9 are as shown in Table 14, and the amino acid sequence is shown in SEQ ID NO: 23.

[Table 14]

| Example 9: Amino Acid Substitutions in Amino Acid Sequence Set Forth in SEQ ID NO: 25 |
|---|
| Q247K R413K R424K Q430K R443K |

Regarding the FAODs of Example 1, Comparative Example 5, and Example 9, the residual activity of each of the FAODs exhibited in a liquid phase under BSA immobilization was measured. One-way ANOVA and post hoc Bonferroni analysis was used for the statistical test. The results are shown in Table 15. The residual activity of Comparative Example 5 was significantly lower than that of Example 1, whereas the residual activity of Example 9 was significantly improved compared to that of Comparative Example 5. Accordingly, it was confirmed that the introduction of the above-described amino acid substitutions can enhance the thermal stability of FAODs exhibited under support immobilization.

[Table 15]

| Mean Value ± Standard Deviation | Residual Activity Under BSA Immobilization (%) |
|---|---|
| Example 1 | 40.5±0.7 [a] |
| Comparative Example 5 | 23.7±1.5 [b] |

(continued)

| Mean Value ± Standard Deviation | Residual Activity Under BSA Immobilization (%) |
|---|---|
| Example 9 | 29.4+2.9 [a] |

The different alphabet indicates that the p-value was less than 0.001 in the statistical test.

While several embodiments and examples of the present disclosure have been described above, these embodiments and examples illustratively describe the present disclosure. For example, the above embodiments are those described in detail so that the present disclosure can be clearly described, and, if necessary, additional modifications may be made to dimensions, configurations, materials, and circuits. Note that the scope of the present disclosure encompasses embodiments in which one or more of the above-described features of the present disclosure are desirably combined. It is intended that the appended claims cover numerous modifications to the embodiments within the technical spirit of the present disclosure. Accordingly, it is to be understood that the embodiments and examples disclosed herein have been presented by way of illustration and should not be considered as limiting the scope of the present disclosure.

REFERENCE SIGNS LIST

10 glycated protein sensor
12 enzyme layer
14 hydrogen peroxide detector
16 detection surface
18 silane coupling agent
20 glycated protein sensor
22 fructosyl amino acid oxidase layer
24 protease layer

**Claims**

1. A fructosyl amino acid oxidase that satisfies one or more selected from (1) to (5) described below:

(1) the fructosyl amino acid oxidase comprises an amino acid sequence set forth in SEQ ID NO: 1 or an amino acid sequence identical to the amino acid sequence set forth in SEQ ID NO: 1 except that one or more amino acids are deleted, substituted, added, and/or inserted;
(2) the fructosyl amino acid oxidase comprises an amino acid sequence that has a homology of 30% or greater to the amino acid sequence described in (1) and which satisfies the conditions described below in (2-1) and (2-2);

(2-1) in an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines, and
(2-2) in an instance where the amino acid sequence and the amino acid sequence set forth in SEQ ID NO: 1 are aligned with each other, amino acids at positions in the amino acid sequence that correspond to positions 430 and/or 443 of the amino acid sequence set forth in SEQ ID NO: 1 are lysines;

(3) the fructosyl amino acid oxidase comprises an amino acid sequence of any one of SEQ ID NOs: 2 to 23 or an amino acid sequence identical to the amino acid sequence of any one of SEQ ID NOs: 2 to 23 except that one or more amino acids are deleted, substituted, added, and/or inserted;
(4) a residual activity exhibited by the fructosyl amino acid oxidase after being treated at a temperature of 48°C while being immobilized on a support is higher than a residual activity exhibited by the fructosyl amino acid oxidase after being treated under the same condition but without being immobilized on a support; and
(5) a residual activity exhibited by the fructosyl amino acid oxidase after being treated at a temperature of 65°C to 80°C for 20 minutes while being immobilized on a support is 70% or greater.

2. The fructosyl amino acid oxidase according to Claim 1, wherein the fructosyl amino acid oxidase satisfies one or more selected from (1) to (3) and satisfies (4) and/or (5).

3. The fructosyl amino acid oxidase according to Claim 2, wherein, regarding (4) and/or (5), the fructosyl amino acid oxidase is immobilized on the support as a result of crosslinking induced by an amine-reactive crosslinking agent.

4. The fructosyl amino acid oxidase according to Claim 3, wherein the fructosyl amino acid oxidase is a recombinant protein.

5. The fructosyl amino acid oxidase according to Claim 1, wherein the fructosyl amino acid oxidase satisfies (2) and further satisfies one or more selected from (2-3) and (2-4) described below:

> (2-3) in an instance where the amino acid sequence and the amino acid sequence set forth in SEQ ID NO: 1 are aligned with each other, amino acids at positions in the amino acid sequence that correspond to positions 309 and/or 413 of the amino acid sequence set forth in SEQ ID NO: 1 are lysines; and
> (2-4) in an instance where the amino acid sequence and the amino acid sequence set forth in SEQ ID NO: 1 are aligned with each other, an amino acid at a position in the amino acid sequence that corresponds to position 424 of the amino acid sequence set forth in SEQ ID NO: 1 is a lysine.

6. The fructosyl amino acid oxidase according to Claim 1, wherein the fructosyl amino acid oxidase satisfies (2) and further satisfies one or more selected from (2-5) and (2-6) described below:

> (2-5) in an instance where the amino acid sequence and the amino acid sequence set forth in SEQ ID NO: 1 are aligned with each other, amino acids at positions in the amino acid sequence that correspond to positions 424, 430, and 443 of the amino acid sequence set forth in SEQ ID NO: 1 are lysines; and
> (2-6) in an instance where the amino acid sequence and the amino acid sequence set forth in SEQ ID NO: 1 are aligned with each other, amino acids at positions in the amino acid sequence that correspond to positions 309 and 413 of the amino acid sequence set forth in SEQ ID NO: 1 are lysines.

7. The fructosyl amino acid oxidase according to Claim 6, wherein the fructosyl amino acid oxidase further satisfies (4).

8. The fructosyl amino acid oxidase according to Claim 7, wherein, regarding (4), the fructosyl amino acid oxidase is immobilized on the support as a result of crosslinking induced by an amine-reactive crosslinking agent.

9. The fructosyl amino acid oxidase according to Claim 8, wherein the amino acid sequence has a homology of 74% or greater to the amino acid sequence set forth in SEQ ID NO: 1.

10. The fructosyl amino acid oxidase according to Claim 5, wherein

> the fructosyl amino acid oxidase satisfies (2-3) and (2-4), and
> in an instance where the amino acid sequence and the amino acid sequence set forth in SEQ ID NO: 1 are aligned with each other, amino acids at positions in the amino acid sequence that correspond to positions 34, 177, 233, 234, and 297 of the amino acid sequence set forth in SEQ ID NO: 1 are lysines.

11. The fructosyl amino acid oxidase according to Claim 10, wherein the amino acid sequence has a homology of 74% or greater to the amino acid sequence set forth in SEQ ID NO: 1.

12. A fructosyl amino acid oxidase, wherein

> the fructosyl amino acid oxidase comprises an amino acid sequence that has a homology of 30% or greater to an amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines, and
> the fructosyl amino acid oxidase satisfies (6-1) and/or (6-2) described below:

> > (6-1) in an instance where the amino acid sequence and the amino acid sequence set forth in SEQ ID NO: 1 are aligned with each other, amino acids at positions in the amino acid sequence that correspond to positions 64, 84, 87, 128, 157, 166, 167, 394, and 397 of the amino acid sequence set forth in SEQ ID NO: 1 are lysines; and
> > (6-2) in an instance where the amino acid sequence and the amino acid sequence set forth in SEQ ID NO: 1 are aligned with each other, amino acids at positions in the amino acid sequence that correspond to positions 112, 137, 152, 247, 254, 255, 267, 277, and 438 of the amino acid sequence set forth in SEQ ID NO: 1 are lysines.

13. A fructosyl amino acid oxidase, wherein

the fructosyl amino acid oxidase comprises an amino acid sequence that has a homology of 30% or greater to an amino acid sequence set forth in SEQ ID NO: 1 and in which, in an instance where the amino acid sequence and the amino acid sequence set forth in SEQ ID NO: 1 are aligned with each other, amino acids at positions in the amino acid sequence that correspond to positions 247 and/or 277 of the amino acid sequence set forth in SEQ ID NO: 1 are lysines, and
the fructosyl amino acid oxidase comprises amino acid substitutions as described below in (7-1) and/or (7-2):

(7-1) in an instance where the amino acid sequence and the amino acid sequence set forth in SEQ ID NO: 1 are aligned with each other, and an amino acid other than lysine is present at a position in the amino acid sequence while a lysine is present at a corresponding position in the amino acid sequence set forth in SEQ ID NO: 1, the amino acid other than lysine is substituted with a lysine; and
(7-2) in an instance where the amino acid sequence and the amino acid sequence set forth in SEQ ID NO: 1 are aligned with each other, and a lysine is present at a position in the amino acid sequence while an amino acid other than lysine is present at a corresponding position in the amino acid sequence set forth in SEQ ID NO: 1, the lysine is substituted with an amino acid identical to the amino acid present at the corresponding position in the amino acid sequence set forth in SEQ ID NO: 1.

14. The fructosyl amino acid oxidase according to Claim 13, wherein a residual activity exhibited by the fructosyl amino acid oxidase after being heat-treated while being immobilized on a support is higher than a residual activity exhibited by a fructosyl amino acid oxidase comprising an amino acid sequence identical to the foregoing amino acid sequence except that the amino acid substitutions as described in (7-1) and (7-2) are not incorporated.

15. The fructosyl amino acid oxidase according to Claim 14, wherein, regarding the immobilization on the support, the fructosyl amino acid oxidase is immobilized on the support as a result of crosslinking induced by an amine-reactive crosslinking agent.

16. The fructosyl amino acid oxidase according to Claim 15, wherein the heat treatment is a treatment at a temperature of 48°C to 80°C.

17. The fructosyl amino acid oxidase according to Claim 14, wherein the position in (7-1) and (7-2) is one or more selected from

positions 56 to 99, 120 to 132, 155 to 171, and 353 to 404 of the amino acid sequence set forth in SEQ ID NO: 1,
positions 100 to 119, 133 to 154, 241 to 288, and 435 to 440 of the amino acid sequence set forth in SEQ ID NO: 1, and
positions 1 to 55, 172 to 240, 289 to 352, 405 to 434, and 441 to 446 of the amino acid sequence set forth in SEQ ID NO: 1.

18. The fructosyl amino acid oxidase according to any one of Claims 1 to 17, wherein the fructosyl amino acid oxidase has a specific reactivity with fructosyl lysine.

19. The fructosyl amino acid oxidase according to Claim 18, wherein, regarding the reactivity, the fructosyl amino acid oxidase has a reactivity with fructosyl valine or a peptide containing fructosyl valine of 20 or less based on the reactivity with fructosyl lysine, which is taken as 100.

20. The fructosyl amino acid oxidase according to Claim 19, wherein, regarding the reactivity, the fructosyl amino acid oxidase has a reactivity with fructosyl glycine of 20 or less based on the reactivity with fructosyl lysine, which is taken as 100.

21. A method for producing a fructosyl amino acid oxidase, the method comprising the step of substituting one or more amino acids in an amino acid sequence of a fructosyl amino acid oxidase with a lysine, the amino acid sequence having a homology of 30% or greater to an amino acid sequence set forth in SEQ ID NO: 1, the one or more amino acids being at one or more positions in the amino acid sequence that correspond to one or more selected from positions 309, 413, 424, 430, and 443 of the amino acid sequence set forth in SEQ ID NO: 1, in an instance where the amino acid sequence and the amino acid sequence set forth in SEQ ID NO: 1 are aligned with each other, wherein a thermal stability, which is exhibited while the fructosyl amino acid oxidase is immobilized on a support, of the fructosyl amino acid oxidase consisting of an amino acid sequence that has undergone the amino acid substitution is higher than a thermal stability, which is exhibited while the fructosyl amino acid oxidase is immobilized on a support, of the fructosyl amino acid oxidase consisting of the amino acid sequence that has not yet undergone the amino acid substitution.

22. A method for producing a fructosyl amino acid oxidase, the method comprising the step of substituting amino acids in an amino acid sequence of a fructosyl amino acid oxidase with a lysine, the amino acid sequence having a homology of 30% or greater to an amino acid sequence set forth in SEQ ID NO: 1, the amino acids being amino acids described below in any one of (8-1) to (8-3), wherein a thermal stability, which is exhibited while the fructosyl amino acid oxidase is immobilized on a support, of the fructosyl amino acid oxidase consisting of an amino acid sequence that has undergone the amino acid substitution is higher than a thermal stability, which is exhibited while the fructosyl amino acid oxidase is immobilized on a support, of the fructosyl amino acid oxidase consisting of the amino acid sequence that has not yet undergone the amino acid substitution:

   (8-1) amino acids at positions in the amino acid sequence that correspond to positions 64, 84, 87, 128, 157, 166, 167, 394, and 397 of the amino acid sequence set forth in SEQ ID NO: 1, in an instance where the amino acid sequence and the amino acid sequence set forth in SEQ ID NO: 1 are aligned with each other;
   (8-2) amino acids at positions in the amino acid sequence that correspond to positions 112, 137, 152, 247, 254, 255, 267, 277, and 438 of the amino acid sequence set forth in SEQ ID NO: 1, in an instance where the amino acid sequence and the amino acid sequence set forth in SEQ ID NO: 1 are aligned with each other; and
   (8-3) amino acids at positions in the amino acid sequence that correspond to positions 34, 177, 233, 234, 297, 309, 413, 424, 430, and 443 of the amino acid sequence set forth in SEQ ID NO: 1, in an instance where the amino acid sequence and the amino acid sequence set forth in SEQ ID NO: 1 are aligned with each other.

23. A method for producing a fructosyl amino acid oxidase, the method comprising the step of performing amino acid substitution as described below in (9-1) and (9-2), on an amino acid sequence of a fructosyl amino acid oxidase, the amino acid sequence having a homology of 30% or greater to an amino acid sequence set forth in SEQ ID NO: 1, wherein a thermal stability, which is exhibited while the fructosyl amino acid oxidase is immobilized on a support, of the fructosyl amino acid oxidase consisting of an amino acid sequence that has undergone the amino acid substitution is higher than a thermal stability, which is exhibited while the fructosyl amino acid oxidase is immobilized on a support, of the fructosyl amino acid oxidase consisting of the amino acid sequence that has not yet undergone the amino acid substitution:

   (9-1) in an instance where the amino acid sequence and the amino acid sequence set forth in SEQ ID NO: 1 are aligned with each other, and an amino acid other than lysine is present at a position in the amino acid sequence while a lysine is present at a corresponding position in the amino acid sequence set forth in SEQ ID NO: 1, the amino acid other than lysine is substituted with a lysine; and
   (9-2) in an instance where the amino acid sequence and the amino acid sequence set forth in SEQ ID NO: 1 are aligned with each other, and a lysine is present at a position in the amino acid sequence while an amino acid other than lysine is present at a corresponding position in the amino acid sequence set forth in SEQ ID NO: 1, the lysine is substituted with an amino acid identical to the amino acid present at the corresponding position in the amino acid sequence set forth in SEQ ID NO: 1.

24. The method for producing a fructosyl amino acid oxidase according to any one of Claims 21 to 23, wherein the thermal stability is a thermal stability exhibited while the fructosyl amino acid oxidase is immobilized on the support as a result of crosslinking induced by an amine-reactive crosslinking agent.

25. The method for producing a fructosyl amino acid oxidase according to Claim 24, wherein

   a residual activity exhibited by the fructosyl amino acid oxidase after being treated at a temperature of 48°C while being immobilized on a support is higher than a residual activity exhibited by the fructosyl amino acid oxidase after being treated under the same condition but without being immobilized on a support; and/or

a residual activity exhibited by the fructosyl amino acid oxidase after being treated at a temperature of 65°C to 80°C for 20 minutes while being immobilized on a support is 70% or greater.

26. The method for producing a fructosyl amino acid oxidase according to Claim 25, wherein the homology is 74% or greater.

27. A glycated protein sensor comprising:

a support; and
a fructosyl amino acid oxidase immobilized on the support, wherein
a residual activity exhibited by the fructosyl amino acid oxidase after being treated at a temperature of 65°C to 80°C for 20 minutes while being immobilized on a support is 70% or greater.

28. A glycated protein sensor comprising:

a support; and
a fructosyl amino acid oxidase immobilized on the support, wherein
the fructosyl amino acid oxidase is the fructosyl amino acid oxidase according to any one of Claims 1 to 17.

29. The glycated protein sensor according to Claim 28, wherein the fructosyl amino acid oxidase is immobilized on the support as a result of crosslinking induced by an amine-reactive crosslinking agent.

30. The glycated protein sensor according to Claim 29, further comprising a hydrogen peroxide detector.

31. The glycated protein sensor according to Claim 30, wherein a support layer and the hydrogen peroxide detector are layered on top of each other, the support layer including the support.

32. A method for measuring a glycated protein, the method comprising detecting a glycated protein by using a reaction of a fructosyl amino acid oxidase immobilized on a support, wherein
the fructosyl amino acid oxidase is the fructosyl amino acid oxidase according to any one of Claims 1 to 17.

FIG. 1

```
                                                          ▽
SEQ ID No:1    1:MASSNLTLTSSILIVGAGTWGCSTALHLARRGYKNVTVLDPHPVPSPIAAGNDINKIMEH 60
SEQ ID No:24   1:MTSSKLTPTSSILIVGAGTWGCSTALHLARRGYKNVTVLDPHPVPSPIAAGNDINKIMEH 60
SEQ ID No:2    1:MATLELNHTTSILIVGAGTWGCSTALHLARRGYRNVKVLDPHPVPSPIAAGNDINKIMEH 60
SEQ ID No:3    1:MATLELNHASSILIVGAGTWGCSTALHLARRGYKNVKVLDPHPVPSPIAAGNDINKIMEH 60
SEQ ID No:25   1:MAPSILSTESSIIVIGAGTWGCSTALHLARRGYKDVTVLDPHPVPSPIAAGNDINKIMEH 60
SEQ ID No:4    1:-MASELSTSSSILIVGAGTWGCSTALHLARRGYKHVTVLDPYPVPSPIAAGNDVNKIMEH 59
SEQ ID No:5    1:-MAGGLGTQSRILILGGGTWGCSTALHLARRGYKNVTVLDAHPIPSPISAGNDVNKIVEQ 59
                .. . *    ..*...*.*****************..*.***..*.****.****.***.*.

SEQ ID No:1   61:REVKASETDPWSIAFSTCTRAALKGWKTDPVFQPYFHETGAIVSGHTPSLIKHIEEHEID 120
SEQ ID No:24  61:REVKASETDPWSIAFSTCTRAALKGWKNDPVFQPYFHETGAIVSGHTASLIKHIQEHEID 120
SEQ ID No:2   61:KELKSPKPDARSIAFATCTRAALKGWKTDPVFQPYFHETGFIVSGHTPALIEHIKTEEID 120
SEQ ID No:3   61:KELKSPKPDARSIAFATCTRAALKGWKTDPVFQPYFHETGFIVSGHTPALIEHIKTEDID 120
SEQ ID No:25  61:SELKDGSSDPRSAAFSTFTRAALKAWKTDPVFQPYFHETGFIISGHTPALIDHIRKDEVE 120
SEQ ID No:4   60:KEVAG------IIAFATCTRAALKGWQTDPVFKPYFHETGFIQAGHTPALIEHIRQNDLY 113
SEQ ID No:5   60:GCFTDG--DDEAAVAQTLLHAANEGWQKDPVFQPYYHDTGYIVAGSSPKAIERLVDREVR 117
                 . .    . ... *...**...*.****.**.*.**.*..*.....*...   ...

                                                ▽
SEQ ID No:1  121:SSDAEFVKLNTAEDFRKTMPPGILTGNFPGWKGWLNKTGAGWIHAKKAMFSAYTEAKRLG 180
SEQ ID No:24 121:SSDAEFIKLNTAEDFRKTMPPGILTGNFPGWKGWLNKTGAGWIHAKKAMFSAYTEAKRLG 180
SEQ ID No:2  121:PSEGDWVALESAEDFRQTMPPGVLTGDFPGWKGWISRVGAGWIHARKAMVSAYNEAQRLG 180
SEQ ID No:3  121:PSEGDWAALESAEDFRQTMPPGVLTGDFPGWKGWISRVGTGWIHARKAMVSAYNEAQRLG 180
SEQ ID No:25 121:PSETNFVKLETAEDFRRTMPPGVLTGDFPGWKGWLHKSGAGWIHAKKAMISAFNEAKRLG 180
SEQ ID No:4  114:PSESDFVKLETAEDFRKTMPPGVLTGDFPGWKGWLMKKNAGWIHARKAMFSAFHEAKRLG 173
SEQ ID No:5  118:HQASSFRKLTTAEDFKGTMPTGVLTGDFPGWEGYYKPSGAGWVHARKALVSAYNEAKRLG 177
                ...  ...*..****. ***.*.***.****.*.. . ..**.**.**. **..**.***

                                        W̅W̅
SEQ ID No:1  181:VTFITGSPEGDVVSLVYENGDVAGARTADGTIHRAEHTILSAGAGSDRLLDFKKQLRPTA 240
SEQ ID No:24 181:VTFITGSPEGDVVSLIYENGDVVGARTADGTVHRADHTILSAGAGSDRLLDFKKQLRPTA 240
SEQ ID No:2  181:VDFVTGSPQGNVESLIYKDADVVGARTADGLSHYADRTILAAGAGSDRLLDFKKQLRPTA 240
SEQ ID No:3  181:VSFVTGSPEGNVESLIYKDGDVVGARTADGLSHYADRTILAAGAGSDRLLDFKKQLRPTA 240
SEQ ID No:25 181:VRFVTGSPEGNVVSLVYEDGDVVGARTADGRVHKAHRTILSAGAGSDSLLDFKKQLRPTA 240
SEQ ID No:4  174:VTFITGSPQGKVVSLVYGQDDVTGARTEDGKVHSADRVIVSAGAGTDRILDMENQLRPTA 233
SEQ ID No:5  178:VNFITGSPEGKVKSLLLEDGDVRGAITADGVEHRADRTILAMGANAPQLLDFENQLRPTA 237
                * *.****.* *.** ....**.**.*.** * *...**.....**...******
```

FIG. 2

```
                 ↓                          ↓              ▽
SEQ ID No:1  241:WTLCHIKMTPEEAK--KYRNLPVLFNVAKGFFMEPDEDKHELKICDEHPGYCNFIPDPKH 298
SEQ ID No:24 241:WTLCHIRMTPDEAK--KYRNLPVLFNVAKGFFMEPDEDNHELKICDEHPGYCNFVPDPKH 298
SEQ ID No:2  241:WTLSHIRMTPEEAD--KYRNLPVLFNVAKGFFMEPDEEKHELKICDEHPGYCNFVVDPDH 298
SEQ ID No:3  241:WTLSHIRMTPEEAE--KYRNLPVLFNVAKGFFMEPDEEKHELKICDEHPGYCNFVVDPDH 298
SEQ ID No:25 241:WTLCHIQMGPEEVK--QYRNLPVLFNIAKGFFMEPDEDKHELKICDEHPGYCNFLPDPNR 298
SEQ ID No:4  234:WTLCHIKMTEEEIKSQRYRNLPVLFNIAKGFFMEPDEDKGELKMCDEHPGYVNWVSDPNR 293
SEQ ID No:5  238:WTLAHIPMTEEETR--LYKNLPVLFNIEKGFFMEPDEDKHELKLCDEHPGYCNWVDQPG- 294
                ***.** *...*..  .*.*******..*********...***.*******.*...*. .

                        ▼
SEQ ID No:1  299:GGEVRSIPFAKHQIPLEAEARARDFLRDTMPHLADRPLSFARICWDADTVDRAFLIDRHP 358
SEQ ID No:24 299:GGEVRSIPFAKHQIPLEAEARARDFLRDTMPHLADRPLSFARICWDADTVDRAFLIDRHP 358
SEQ ID No:2  299:QGEKRSIPFAKEQIPLEAEARARDFLRDTMPHLADRPFSFARICWDADTVDRAFLIDRHP 358
SEQ ID No:3  299:QGEKRSIPFAKEQIPLEAEARARDFLRDTMPHLADRPFSFARICWDADTVDRAFLIDRHP 358
SEQ ID No:25 299:PGQEKSVPFAKHQIPLEAEARARDFLHDTMPHLADRPLSFARICWDADTPDRAFLIDRHP 358
SEQ ID No:4  294:PGEKRSIPFAKEQIPREAERRAREFLKDTMPHLADRPLVFARICWDADTPDRFFLIDYHP 353
SEQ ID No:5  295:SALPQSVPVAKHEVPASAERRMRDFLREIMPHLADRPFTFARMCWCADTRNRAFLITYHP 354
                .. .*.*.**...*..**.*.*.**...*******..***.**.***..*.***..**

                                                            ▼
SEQ ID No:1  359:EYRSLLLAVGGSGNGAMQMSTIGGFITDALEGHLQKELKHALRWRPEIATERDWKDTQNR 418
SEQ ID No:24 359:EYRSLLLAVGGSGNGAMQMPTIGGFIADALEGNLQKELKHALRWRPEIAAQRDWKDTQNR 418
SEQ ID No:2  359:EYASLVVAVGGSGNGAMQMPTIGGFISDTLEGKLQKELKDVVRWRPETAIHRDWKSTQNR 418
SEQ ID No:3  359:EYASLVVAVGGSGNGAMQMPTIGGFISDVLEGKLQKELKDVVRWRPEIAIRRDWKSTQNR 418
SEQ ID No:25 359:EHPSLLVAVGGSGNGAMQMPTIGGFIADALESKLQKEVKDIVRWRPETAVDRDWRATQNR 418
SEQ ID No:4  354:RYPSLLLACGGSGNGAMQMPSIGGFIADALEGRLQEELKDVCRWRPETAVGRDWKSTQGR 413
SEQ ID No:5  355:KHPSLVVASGDSGHGFMHIPSIGGFIVDCMENKLQPNFAKSWRWRPETTRDFWGRDVLDR 414
                .. **..*.*.**.*.*....***** *.*.**..... *****.. .... ...*

                       ▼    ↓  ↓
SEQ ID No:1  419:FGGPNKVMDFQKVGENEWTKIGDD-KCRL                           446
SEQ ID No:24 419:FGGPNKVMDFQKVGENEWTKIGD--KSRL                           445
SEQ ID No:2  419:FGGPDRLMDFQDVKDDEWTRIGEDMASRL                           447
SEQ ID No:3  419:FGGPDRLMDFQDVKDDEWTSIGEEMASRL                           447
SEQ ID No:25 419:FGGPDRIMDFQQVGEDQWTKIGESRGP--                           445
SEQ ID No:4  414:FGGPDKVMDFQEVGENEWTRVGKE--SLL                           440
SEQ ID No:5  415:FGAGNKMLDVNETREEGWTNVNATPLTVA                           443
                **.... .*.. ... .** ..    ...
```

FIG. 3

10

12

18

14    16

FIG. 4

FIG. 5

FIG. 6

RESIDUAL ACTIVITY

FIG. 7

FIG. 8

FIG. 9

(A)

RESIDUAL ACTIVITY (%)

Bar: S.D.; **: $p < 0.01$

(B)

RESIDUAL ACTIVITY (%)

Bar: S.D.; **: $p < 0.01$

<table>
<tr><td colspan="2" align="center">INTERNATIONAL SEARCH REPORT</td><td>International application No.<br>**PCT/JP2023/001930**</td></tr>
</table>

**A. CLASSIFICATION OF SUBJECT MATTER**

*C12N 9/02*(2006.01)i; *C12M 1/34*(2006.01)i; *C12Q 1/26*(2006.01)i; *G01N 27/327*(2006.01)i; *G01N 27/416*(2006.01)i
FI:  C12N9/02 ZNA; C12M1/34 E; C12Q1/26; G01N27/327 353F; G01N27/416 336G

According to International Patent Classification (IPC) or to both national classification and IPC

**B. FIELDS SEARCHED**

Minimum documentation searched (classification system followed by classification symbols)

C12N9/02; C12M1/34; C12Q1/26; G01N27/327; G01N27/416

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); GenBank/EMBL/DDBJ/GeneSeq; UniProt/GeneSeq; SwissProt/GeneSeq

**C. DOCUMENTS CONSIDERED TO BE RELEVANT**

| Category* | Citation of document, with indication, where appropriate, of the relevant passages | Relevant to claim No. |
|---|---|---|
| X | UNIPROTKB [online]. FAD dependent oxidoreductase. UniProtKB ACCESSION No. A0A5N6TC99. Last updated: 22 April 2020, [retrieved on 07 March 2023], <URL: http://www.uniprot.org/uniprotkb/A0A5N6TC99/entry><br>especially, pp. 1-4 | 1-20 |
| Y | | 21-32 |
| X | UNIPROTKB [online]. FAD dependent oxidoreductase. UniProtKB ACCESSION No.A0A5N6UZA0, Last updated: 22 April 2020, [retrieved on 07 March 2023], <URL: http://www.uniprot.org/uniprotkb/A0A5N6UZA0/entry><br>especially, pp. 1-4 | 1-20 |
| Y | | 21-32 |
| X | JP 2013-500729 A (F. HOFFMANN-LA ROCHE AG) 10 January 2013 (2013-01-10)<br>SEQ ID NO: 10, paragraph [0057] | 1-20 |
| Y | | 21-32 |

☑ Further documents are listed in the continuation of Box C.     ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| | International application No. |
|---|---|
| | **PCT/JP2023/001930** |

**C.    DOCUMENTS CONSIDERED TO BE RELEVANT**

| Category* | Citation of document, with indication, where appropriate, of the relevant passages | Relevant to claim No. |
|---|---|---|
| X | WO 2015/060431 A1 (KIKKOMAN CORPORATION) 30 April 2015 (2015-04-30) in particular, SEQ ID NO: 127 | 1-20 |
| Y | | 21-32 |
| X | JP 2009-000084 A (TOYO BOSEKI) 08 January 2009 (2009-01-08) claims, examples, SEQ ID NO: 2 | 1-20 |
| Y | | 21-32 |
| Y | JP 2010-233502 A (TOYO BOSEKI) 21 October 2010 (2010-10-21) claims, examples | 21-32 |
| Y | WO 2019/221264 A1 (PROVIGATE INC) 21 November 2019 (2019-11-21) claims, examples | 21-32 |

Form PCT/ISA/210 (second sheet) (January 2015)

**INTERNATIONAL SEARCH REPORT**

International application No.

**PCT/JP2023/001930**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

   a.  ☑ forming part of the international application as filed:

       ☑ in the form of an Annex C/ST.25 text file.

       ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

       ☐ in the form of an Annex C/ST.25 text file (Rule 13*ter*.1(a)).

       ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

   "The form of Annex C/ST.25 text file" above shall read as "the form of ST.26.".

Form PCT/ISA/210 (continuation of first sheet) (January 2015)

## INTERNATIONAL SEARCH REPORT
### Information on patent family members

International application No.

**PCT/JP2023/001930**

| Patent document cited in search report | | | Publication date (day/month/year) | Patent family member(s) | | | Publication date (day/month/year) |
|---|---|---|---|---|---|---|---|
| JP | 2013-500729 | A | 10 January 2013 | US | 2012/0208226 | A1 | |
| | | | | SEQ ID NO. 10, paragraph [0064] | | | |
| | | | | WO | 2011/015326 | A2 | |
| | | | | EP | 2287295 | A1 | |
| WO | 2015/060431 | A1 | 30 April 2015 | US | 2016/0274129 | A1 | |
| | | | | especially, SEQ ID NO. 127 | | | |
| | | | | EP | 3061829 | A1 | |
| JP | 2009-000084 | A | 08 January 2009 | (Family: none) | | | |
| JP | 2010-233502 | A | 21 October 2010 | (Family: none) | | | |
| WO | 2019/221264 | A1 | 21 November 2019 | US | 2021/0223200 | A1 | |
| | | | | claims, examples | | | |
| | | | | EP | 3812450 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014183786 A
- JP 2011229526 A
- JP 2010233502 A

- JP 6504586 B
- JP 2013176351 A
- JP 2009000084 A